# EUROPEAN PATENT SPECIFICATION

(11) **EP 4 356 923 B1**
(45) Date of publication and mention of the grant of the patent: **29.10.2025**
(21) Application number: 23205060.9
(22) Date of filing: 20.10.2023
(51) Int. Cl.: A61K 38/45, A61P 25/00, A61P 25/28, C12N 9/12, C12N 15/63

(54) **PKMYT1 FOR USE IN REGENERATIVE MEDICINE**
PKMYT1 ZUR VERWENDUNG IN DER REGENERATIVEN MEDIZIN
PKMYT1 POUR UTILISATION EN MÉDECINE RÉGÉNÉRATIVE

(30) Priority: 20.10.2022 PT 2022118269
(43) Date of publication of application: 24.04.2024
(73) Proprietor: Universidade de Aveiro, 3810-193 Aveiro (PT)
(72) Inventor: MOREIRA PINTO VIEIRA, SANDRA ISABEL, 3810-193 AVEIRO (PT); DIAS CORREIA, PATRÍCIA MARIA, 3810-193 AVEIRO (PT); MAMEDE DE SOUSA, BÁRBARA, 3810-193 AVEIRO (PT); DA SILVA FIGUEIREDO, JOSÉ GABRIEL, 3810-193 AVEIRO (PT)
(74) Representative: Patentree

(56) References cited:
- US-A1- 2004 077 049
- US-A1- 2005 266 409
- US-A1- 2022 025 369
- ASQUITH CHRISTOPHER R M ET AL: "PKMYT1: a forgotten member of the WEE1 family", NATURE REVIEWS DRUG DISCOVERY, NATURE PUBLISHING GROUP, GB, 26 November 2019 (2019-11-26), XP037049359, ISSN: 1474-1776, [retrieved on 20191126], DOI: 10.1038/D41573-019-00202-9
- MOLINA ANGIE ET AL: "Playing with the cell cycle to build the spinal cord", DEVELOPMENTAL BIOLOGY, vol. 432, no. 1, 27 December 2016 (2016-12-27), pages 14 - 23, XP085262849, ISSN: 0012-1606, DOI: 10.1016/J.YDBIO.2016.12.022
- NIE BANG-XU ET AL: "Inhibition of CDK1 attenuates neuronal apoptosis and autophagy and confers neuroprotection after chronic spinal cord injury in vivo", vol. 119, 1 January 2022 (2022-01-01), GB, pages 102053, XP093077589, ISSN: 0891-0618, Retrieved from the Internet <URL:https://pdf.sciencedirectassets.com/271026/1-s2.0-S0891061821X00087/1-s2.0-S0891061821001368/main.pdf?X-Amz-Security-Token=IQoJb3JpZ2luX2VjEAIaCXVzLWVhc3QtMSJHMEUCIBiUy1Btwru4ojLPDmBwHOa0oKfhKNbmLcjrU4XnlDycAiEA1xUbOGBJQlJprwohU1n9cp3XI4ErWacqIc9cMY+twzEqvAUIy///////////ARAFGgwwNTkwMDM1NDY4NjUiDNu2m> DOI: 10.1016/j.jchemneu.2021.102053
- JOS� GABRIEL DA SILVA FIGUEIREDO: "PKMYT1 and other potential spinal cord regeneration-associated genes - PKMYT1 e outros genes potencialmente associados � regenera��o da medula espinhal", 1 January 2021 (2021-01-01), XP093112133, Retrieved from the Internet <URL:https://opac.ua.pt/cgi-bin/koha/opac-MARCdetail.pl?biblionumber=301933>

## Description

### TECHNICAL FIELD

The present disclosure relates to isolated or artificial nucleotide sequences encoding the protein kinase, membrane-associated tyrosine/threonine 1 (PKMYT1), wherein the sequence encoding the protein PKMYT1 is identical to a sequence selected from a list consisting of: SEQ. ID. 1, SEQ. ID. 6, SEQ. ID. 7, for use in the treatment or regeneration of neurons following traumatic injuries on the central or peripheral nervous systems, preferably in the treatment of spinal cord injury. Furthermore, the present invention is also related to a vector comprising such sequence, a host cell comprising such vector, a protein PKMYT1, or a composition thereof, said vector, host cell, protein and composition for use in the treatment or regeneration of neurons following traumatic injuries on the central or peripheral nervous systems, preferably in the treatment of spinal cord injury.

### BACKGROUND

The neuroregenerative capacity of the Central Nervous System (CNS) is very low in mammals and declines even further into adulthood. In general, CNS neurodegeneration or trauma induces a persistent inhibitory environment around the injured zone that hinders effective neuroregeneration¹⁻³. As such, spinal cord injury (SCI) has no treatment and is highly deleterious to the patients, their caregivers and the Healthcare system. Further, although the lesioned nerves in the peripheral nervous system (PNS) can significantly spontaneously regenerate small defects, the regeneration of severe peripheral lesions is less effective and needs support. Genetic and molecular combined therapies may promote a regenerative response, alone or when combined with cellular and biomaterial-based therapies aiming to ameliorate SCl⁴. Identification of functional target genes and signaling pathways, capable of improving regeneration at the lesion site when modulated, is one of the main challenges. One strategy is to search for potential neuronal regeneration-associated genes (RAGs) in transcriptomic studies of lesioned tissues from vertebrates that naturally regenerate the spinal cord, from artificially manipulated rodents with some improved recovery, and from PNS regenerating mammalian neurons.

PKMYT1 (protein kinase, membrane-associated tyrosine/threonine 1, previously referred to as *MYT1;* UniProt Accession Number Q99640) is a serine/threonine kinase of the WEE kinase family, which also includes WEE1/WEE1A and WEE2/WEE1B kinases. It has been associated with cell cycle regulation⁵ and is widely expressed in most human tissues at low levels, except for those with high proliferative properties, like the bone marrow, lymphoid tissue and testis, where its expression is highly enriched to regulate cell division. At the cellular level, PKMYT1 is abundantly found in the Golgi membrane, but also in the membrane of the endoplasmic reticulum (ER), nucleus and nucleoli, whereas WEE1-2 are nuclear kinases⁶.

WEE family of kinases are fundamental for the G2-to-meiosis/mitosis (M) checkpoint of the cell cycle, which is needed to secure genomic integrity before cellular division. The trigger factor of G2/M transition is the nuclear accumulation of the CDK1 kinase (also known as CDC2), complexed with its regulatory subunit, the cyclin B^{5,7}. At the G2/M stage, WEE/PKMYT1 kinases inactivate CDK1 by phosphorylation, maintaining the CDK1-cyclin B complex in a low activity state, together with the alteration of CDK1's nucleus-cytoplasm trafficking^{5,6}. In particular, PKMYT1 is extra-nuclearly localized and when it phosphorylates CDK1 at the Thr14 and Tyr15 residues, also causes CDK1 retention in the cytoplasm⁵. CDK1 activity results from the balance between WEE kinases and CDC25 phosphatases, which rapidly dephosphorylate CDK1 if no DNA damage is detected, in a set of complex regulatory feedback loop mechanisms⁷. Indeed, CDK1 is capable of phosphorylating PKMYT1 and, although this is not sufficient to silence PKMYT1 activity by itself⁸, it serves as a tag for subsequent PKMYT1 targeting by other regulatory kinases⁹, and is a part of the 'CDK1 auto-amplification loop'. During meiosis in *Xenopus laevis* oocytes, Akt kinase and the ribosomal protein S6 kinase alpha (RPS6KA, also referred to as p90^{rsk}; the downstream effector kinase of the Mos-MAPK signaling pathway) both inhibit PKMYT1 to allow the G2-to-meiosis transition⁹. In subsequent embryonic divisions and in somatic cells' mitosis, Polo-like kinase 1 (PLK1) is the main regulator of PKMYT1, strongly suppressing its activity by direct phosphorylation. During G2 and prometaphase, PKMYT1 can also be inhibited by MAP2K1. PKMYT1 autophosphorylation seems also possible, and proposed as a prerequisite and/or trigger for subsequent PKMYT1 phosphorylation and consequent inactivation. During the M phase of mitotic and meiotic cell cycles, PKMYT1 activity is down-regulated by its hyperphosphorylation. The timing and spatial control of all these intertwining components in the cell cycle are thus crucial for triggering cell division, genomic stability and to define whether the cell survives following divisions or not¹⁰.

In sum, PKMYT1's main function appears to be related to the cell cycle, being active (dephosphorylated) in pre-mitotic cells, where it promotes cell survival, and inactive (phosphorylated) during cell division (mitosis or meiosis). As such, PKMYT1 has been mainly related to cancer. Commonly, cancer cells have a faulty G1 checkpoint and the G2 phase checkpoint is essential for their survival. PKMYT1 and WEE1 are both frequently found to be overexpressed in various cancer types, possibly to maintain cell genomic stability under rapid cell proliferation¹¹. Therefore, emerging anti-cancer therapies target PKMYT1 down-regulation to make cancer cells more unstable and predisposed to apoptosis¹¹, which will be more sensitive to available treatments (Patents: WO2021195782A1, US6448272B1) (CA2892361A1, US20050266409A1, JP4663634B2, US20050249666A1). Besides regulation of cell proliferation, other roles have been suggested for PKMYT1, including taking part in the regulation of the Golgi apparatus and ER membrane reassembly following mitosis⁵. Additionally, it has been associated with development and morphogenesis. PKMYT1 extends the duration of a zygote's first cycle, resulting in a long G2 phase potentially required for preparing events that establish dorsal cell fates. Ectopic expression of both PKMYT1 and WEE1 in *Drosophila melanogaster* disrupts the pattern formation during eye development, by hindering the second mitotic wave. Authors suggest that PKMYT1 could be a downstream target of Notch, since mutations affecting this signaling pathway resulted in similar phenotypes in eye development. Further, when PKMYT1 was silenced in non-small cell lung carcinoma cells, the expression levels of Notch1, p21, and Hes1 were downregulated. The Notch signaling pathway is involved in cell proliferation, tissue topographic organization, chromatin organization and in the development of the nervous system. There is also a possible link between PKMYT1 and the β-catenin/TCF signaling pathway, involved in gene transcription for cell proliferation and differentiation, via PKMYT1 binding and inactivation of GSK3β.

Asquith Christopher R M et al (PKMYT1: forgotten member of the WEE1 family", Nature Reviews Drug Discovery, Nature Publishing Group, GB, 26 November 2019 (2019-11-26), XP037049359, ISSN: 1474-1776, doi: 10.1038/D41573-019-00202-9) disclose WEE1 the founding member of the WEE kinase family, and his implication in over a dozen cancer types, leading to extensive efforts to develop inhibitors. For example, adavosertib (AZD1775), a leading selective WEE1 inhibitor, has been tested as a single agent and in combination in more than 50 clinical trials in patients with cancer. So far, however, another member of the family, PKMYT1 has been largely ignored, although it shares a high degree of functional redundancy with WEE1 and so could also represent a promising and tractable target.

US 2005/266409 A1 disclose Compositions and methods for diagnosing, preventing, and treating cancers. In one embodiment, genes differentially expressed in colon, lung, breast and prostate cancer tissues relative to corresponding cancer-free tissues are identified. These genes or their products can be used as markers for the detection of respective cancers. Modulators of these genes or their products can be used for the treatment or prevention of respective cancers.

US 2022/025369 A1 discloses a purified RNA comprising at least one coding sequence, wherein the at least one coding sequence encodes a peptide or protein comprising a therapeutic protein, wherein the encoded peptide or protein comprises a therapeutic protein or a fragment or variant thereof, selected from a group of therapeutic proteins or a fragment or variant thereof.

Molina Angie et al ("Playing with the cell cycle to build the spinal cord", DEVELOPMENTAL BIOLOGY, vol. 432, no. 1, 27 December 2016 (2016-12-27), pages 14-23, XP085262849, ISSN: 0012-1606, doi: 10.1016/J.YDBIO.2016.12.022) disclose the interplay between the cell cycle and cellular and molecular events governing spinal cord development. The existing links between the cell cycle and interkinetic nuclear migration (INM) are described. It is also shown how the different morphogens patterning the neural tube also regulate the cell cycle machinery to coordinate proliferation and patterning.

Nie Bang-Xu et al ("Inhibition of CDK1 attenuates neuronal apoptosis and autophagy and confers neuroprotection after chronic spinal cord injury in vivo", JOURNAL OF CHEMICAL NEUROANATOMY, vol. 119 1 January 2022 (2022-01-01), page 102053, XP093077589, GB ISSN: 0891-0618, doi: 10.1016/j.jchemneu.2021.102053) disclose the role of CDK1 in CSCI in a rat model. The CSCI model was established by screw compression using the cervical anterior approach for twelve weeks. The neurological function of the rats was evaluated using the neurological severity scores (NSS) and motor evoked potentials (MEPs). It is concluded that CDK1 downregulation suppressed the activation of anterior horn astrocytes and microglia, promoted motor neuron repair, and inhibited neurons apoptosis and autophagy to promote the recovery of motor function after spinal cord injury.

US 2004/077049 A1 discloses reagents and methods of regulating a human WEE1-like serine/threonine protein kinase, and also a pharmaceutical composition comprising a reagent which specifically binds to a polypeptide comprising a specific amino acid sequence and a pharmaceutically acceptable carrier.

José Gabriel Da Silva Figueiredo, 'PKMYT1 and other potential spinal cord regeneration-associated genes', 1 January 2021 (2021-01-01, XPO93112133), is only the title of a thesis. No sequences are disclosed, and no description, abstract, or additional information is provided.

These facts are disclosed in order to illustrate the technical problem addressed by the present disclosure.

### GENERAL DESCRIPTION

The invention is defined in the claims. The present disclosure relates to isolated or artificial nucleotide sequences encoding the proteir kinase membrane-associated tyrosine/threonine 1 (PKMYT1), wherein the sequence encoding the protein PKMYT1 is identical to a sequence selected from a list consisting of: SEQ. ID. 1, SEQ. ID. 6, SEQ. ID. 7, for use in the treatment or regeneration of neurons following traumatic injuries on the central or peripheral nervous systems, preferably in the treatment of spinal cord injury. Furthermore, the present invention is also related to a vector comprising such sequence, a host cell comprising such vector, a protein PKMYT1, or a composition thereof, said vector, host cell, protein and composition for use in the treatment or regeneration of neurons following traumatic injuries on the central or peripheral nervous systems, preferably in the treatment of spinal cord injury.

In a gain-in-function experiment performed, it was surprisingly found that the mean neuritic length was significantly higher in the PKMYT1 up-regulated cells compared to control cells. Further, neurites from PKMYT1 up-regulated cells were comparatively longer since 61% of them were at least 20 µm long, 42% were at least 30 µm long, and 35% were at least 35 µm long, contrasting with the control cells (49%, 34% and 30%, respectively).

In a comparative analysis of PKMYT1 transcripts mRNA levels, in neurons dissected by us from rats following injury in the sciatic nerve (regenerative) or the spinal cord (non-regenerative), neurons under regenerative conditions significantly increased the expression of PKMYT1 by 7.21-fold, when compared to basal levels (sham-operated animals), while the same neurons under non-regenerative conditions did not significantly altered PKMYT1 expression relatively to sham-operated controls.

PKMYT1 is known by the one skilled in the art by several names: Protein Kinase, Membrane Associated Tyrosine/Threonine 1; MYT1; Membrane-Associated Tyrosine- And Threonine-Specific Cdc2-Inhibitory Kinase; PPP1R126; Protein Phosphatase 1, Regulatory Subunit 126; Myt1 Kinase; EC 2.7.11.1.

PKMYT1 gene can be identified by the primary accession numbers NCBI Gene ID 9088; Ensembl ENSG00000127564; MIM:602474; AllianceGenome:HGNC:29650; CCDS10486.1 (NCBI database). This information is available in database source NCBI.

PKMYT1 transcript variants can be identified by the primary accession numbers NCBI Reference sequences NM_004203.5 (SEQ. ID. 6; NM_182687.3; NM_001258450.2; NM_001258451.2. This information is available in database source NCBI.

PKMYT1 protein can be identified by the primary accession number Q99640, including its version Q99640.1 (SEQ. ID. 8) corresponding to the longest isoform (isoform 1), or primary accession number NP_004194.3, or by the secondary accessions B3KUN8; B4DXD4; D3DUA4; F8W164; I3L1V2; 014731; Q7LE24; Q8TCM9. This information is available in database source UniProtKB/Swiss-Prot and database source NCBI.

PKMYT1 protein can also be identified by its isoform 2 (Q99640-2, SEQ. ID. 9), isoform 3 (Q99640-3, SEQ. ID. 10) and isoform 4 (Q99640-4, SEQ. ID. 11).

In an embodiment, the main activity of PKMYT1 is ascribed to its kinase domain, that can be identified by the primary identity references InterPro: IPR000719; Pfam: PF00069; Prosite: PS50011. The kinase domain of the PKMYT1 isoform 1 (accession number Q99640.1 of the UniprotKB/Swiss-Prot database), can be found at amino acid residues number 110-359. This information is available in database source InterPro.

An aspect of the present disclosure relates to an isolated or artificial nucleotide sequence encoding the protein PKMYT1, wherein the sequence encoding the protein PKMYT1 is identical to a sequence selected from a list consisting of: SEQ. ID. 1, SEQ. ID. 6, SEQ. ID. 7; for use in the treatment or regeneration of neurons following traumatic injuries on the central or peripheral nervous system; preferably central nervous system.

In a preferred embodiment, the sequence encoding the protein PKMYT1 is identical to a sequence selected from a list consisting of: SEQ. ID. 6, SEQ. ID. 7.

In a preferred embodiment, the sequence encoding the protein PKMYT1 is identical to the sequence SEQ. ID. 7.

In an embodiment, the isolated or artificial nucleotide sequences herein described are used in the treatment or regeneration of neurons following traumatic injuries on the central nervous systems. In a preferred embodiment, the isolated or artificial nucleotide sequences herein described are used in the treatment of spinal cord injury.

In an embodiment, the isolated or artificial nucleotide sequences are administered directly by in situ injection, by intravascular injection, intravenous injection, intranasal injection, intraventricular injection or intrathecal injection means; preferably by in situ injection.

Another aspect of the present invention relates to a vector or construct comprising the isolated or artificial nucleotide sequences herein described for use in the treatment or regeneration of neurons following traumatic injuries on the central or peripheral nervous system; preferably for use in the treatment of spinal cord injury.

In an embodiment, the vector is selected from the group consisting of: plasmid, liposome, dendrimer, nanoparticle vector, virus vector, preferably adenovirus, lentivirus, retrovirus, herpesvirus and Adeno-Associated Virus; preferably Adeno-Associated Virus or plasmid.

Another aspect of the present disclosure relates to a host cell comprising the vector herein described for use in the treatment or regeneration of neurons following traumatic injuries on the central or peripheral nervous system; preferably central nervous systems; more preferably for use in the treatment of spinal cord injury.

Another aspect of the present disclosure relates to a protein PKMYT1 comprising an amino acid sequence identical to a sequence selected from a list consisting of: SEQ. ID. 8; SEQ. ID. 9; SEQ. ID. 10, SEQ. ID. 11, or mixtures thereof, for use in the treatment or regeneration of neurons following traumatic injuries on the central or peripheral nervous system; preferably central nervous systems; more preferably for use in the treatment of spinal cord injury.

In a preferred embodiment, the amino acid sequence is identical to the sequence SEQ. ID. 8.

Another aspect of the present disclosure relates to a pharmaceutical composition comprising a therapeutically effective amount of an isolated or artificial nucleotide sequence as herein described, or a vector as herein described, or a host cell as herein described , or a protein as herein described, or combinations thereof, for use in the treatment or regeneration of neurons following traumatic injuries on the central or peripheral nervous system; preferably central nervous systems; more preferably for use in the treatment of spinal cord injury.

Another aspect of the present disclosure relates to a liposome, exosome or nanoparticle comprising an isolated or artificial nucleotide sequence as herein described, or a host cell as herein describe, or a protein as herein described, or combinations thereof; for use in the treatment or regeneration of neurons following traumatic injuries on the central or peripheral nervous system; preferably central nervous systems; more preferably for use in the treatment of spinal cord injury.

Another aspect of the present disclosure relates to a kit comprising an isolated or artificial nucleotide sequence as herein described, or a vector as herein described, or a host cell as herein described, or a protein as herein described, or combinations thereof, for use in the treatment or regeneration of neurons following traumatic injuries on the central or peripheral nervous system; preferably central nervous systems; more preferably for use in the treatment of spinal cord injury.

In an embodiment, the sequence is PKMYT1 cDNA of isoform 1 (represented by database accession number NM_004203.5).

In an embodiment, the isolated or artificial nucleotide sequence of the present disclosure may be used in the treatment of injuries inflicted on the central nervous systems.

In an embodiment, the isolated or artificial nucleotide sequence of the present disclosure may be used in the treatment of injuries inflicted on the peripheral nervous systems.

In an embodiment, the isolated or artificial nucleotide sequence of the present disclosure may be used in the treatment of spinal cord injury.

In an embodiment, the sequences referred in the present disclosure can be administered directly into the tissue to treat, *in situ*; preferably into cells of the spinal cord.

In an embodiment, the sequences referred in the present disclosure can be administered by intravascular, intravenous, intranasal, intraventricular or intrathecal injection means.

Another aspect of the present disclosure relates to a vector or construct comprising an isolated or artificial nucleotide sequence herein described for use in the treatment or regeneration of neurons following traumatic injuries on the central or peripheral nervous system; preferably for use in the treatment of spinal cord injury.

In an embodiment, the vector is selected from the group of non-viral vectors, selected from the group of plasmid, liposome, dendrimer, or nanoparticle vector; preferably a plasmid.

In the context of the present disclosure, "G, "C", "A," "T," and "U", each generally stand for a nucleotide that contains guanine, cytosine, adenine, thymidine, and uracil as a base, respectively.

Methods for the alignment of sequences for comparison are well known in the art, such methods include GAP, BESTFIT, BLAST, Clustal Omega. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Clustal Omega is a multiple sequence alignment program that uses seeded guide trees and HMM profile-profile techniques to generate alignments between three or more sequences (Sievers F, Wilm A, Dineen D, et al. (2011) Molecular Systems Biology 7:539), and it is publicly available through EMBL-EBI services. Minor manual editing may be performed to optimise alignment between conserved motifs, as would be apparent to a person skilled in the art. The amino acid sequence identity values, which are indicated in the present subject matter as a percentage, were determined over the entire amino acid sequence, using Clustal Omega with the default parameters.

### BRIEF DESCRIPTION OF THE DRAWINGS

The following figures provide preferred embodiments for illustrating the disclosure and should not be seen as limiting the scope of invention.

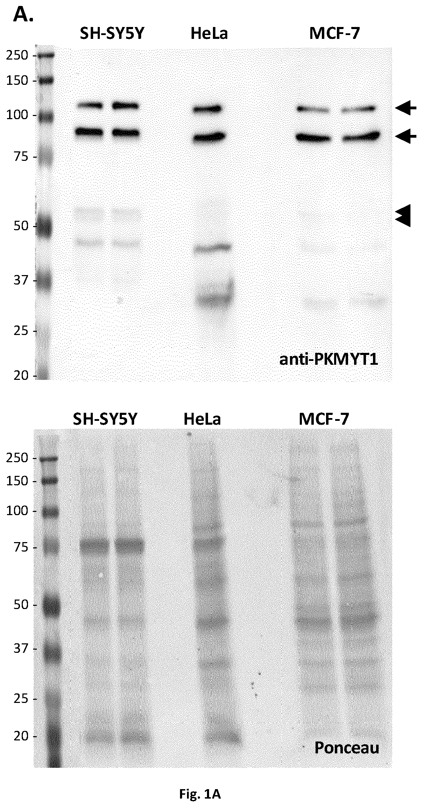

**Figure 1A****: PKMYT1 expression in different cell types, by immunoblot procedures.** Immunoblot analyses of human PKMYT1 endogenous expression in lysates of different cell lines grown for 48h. Upper panel: anti-human PKMYT1 immunoblot; lower panel: pre-staining with Ponceau S as loading control. Molecular weight standard is indicated to the left.

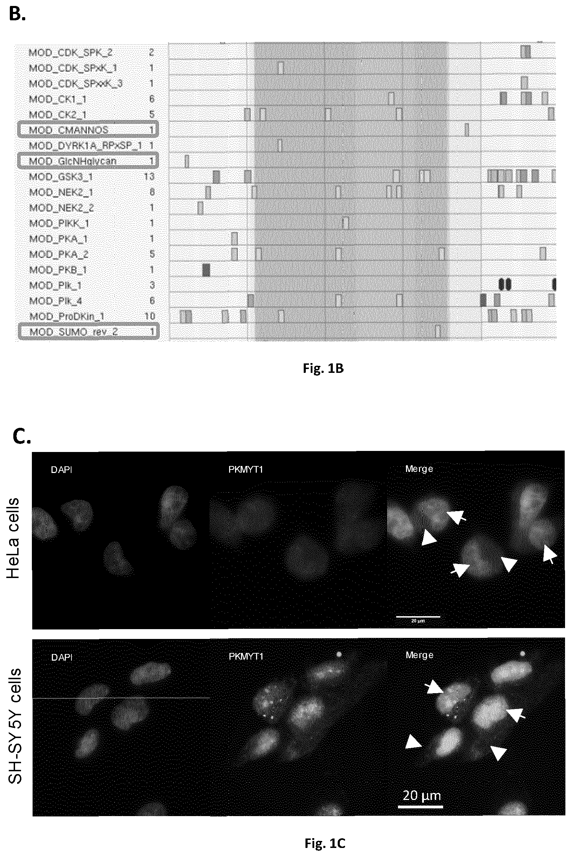

**Figure 1B****: Functional modification motifs of the PKMYT1 protein.** Highlighted are C-Mannosylation ('MOD_CMANNOS') and Glycosaminoglycan attachment ('MOD_GlcNHglycan') modifications, known to highly increase a protein's molecular weight. SUMOylation ('MOD_SUMO_rev_2') also increases a protein molecular weight by 11-12 kDa. Retrieved by the ELM software tool.
**Figure 1C****: PKMYT1 subcellular location in two different cell types.** Epifluorescence microphotographs of the cellular location of PKMYT1, basally expressed in HeLa and SH-SY5Y cells. Upper panel: HeLa cells. Lower panel: SH-SY5Y cells. PKMYT1 is labelled in red in HeLa cells, and in green in SH-SY5Y cells, and appears at the cells nuclei (arrows) and cytoplasm (arrowheads). Nuclei were counterstained with DAPI (in blue). Bar = 20 µm.

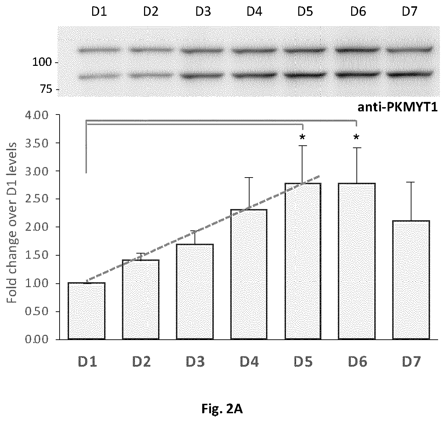

**Figure 2A****: Profile of PKMYT1 protein levels throughout SH-SY5Y proliferation.** Immunoblot analyses of the endogenous expression of human PKMYT1 in SH-SY5Y neuronal-like cells, with time in a proliferative culture; SH-SY5Y cells were seeded at ~4.0 x 10⁴/cm² on day ('D') 0 and cells collected at every day for 1 week (D1-D7); 100% confluency was achieved around D5-6; dashed blue line: linear trendline during the log phase (Y=0.44X+1). Results are presented as averaged fold change + standard deviation. Fold changes were calculated over the PKMYT1 levels at 24 h after seeding (D1, taken as 1.0). N = 3 replicates. Statistically significant differences were analysed using one-way ANOVA. Statistic symbols: **P* < 0.05.

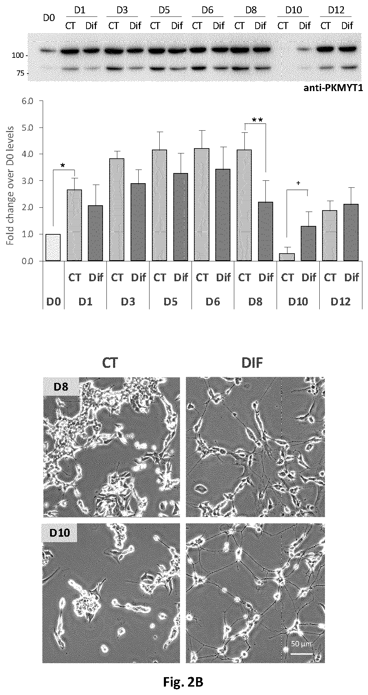

**Figure 2B****: Profile of PKMYT1 protein levels throughout SH-SY5Y neuronal differentiation.** Immunoblot analyses of the endogenous expression of human PKMYT1 in SH-SY5Y neuronal-like cells, during a neuronal differentiation program. SH-SY5Y cells were seeded at ^{~}3.0 x 10⁴/cm² and treated with 10 µM RA (medium with 1% FBS) from D0-D5, when medium was changed to serum-free medium with 10 ng/mL brain-derived neurotrophic factor (BDNF) (D6-D12). Molecular weight standard is presented to the left. Results are presented as averaged fold change + standard deviation. Fold changes were calculated over the PKMYT1 levels at 24 h after seeding (D0, taken as 1.0). N = 3 replicates. Statistically significant differences, analysed using two-way ANOVA ('*') or student t-test ('+'), are presented. Statistic symbols: */⁺*P* < 0.05; ***P <* 0.01. Representative phase contrast microphotographs are presented. Bar = 50 µm.

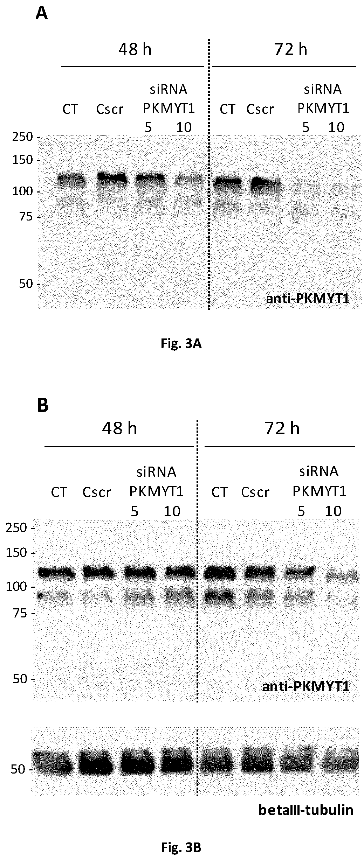

**Figure 3A** - **3D****: Effects of PKMYT1 knockdown on neuronal-like cells' metabolic rate and cell number. A** and **B:** Immunoblots of PKMYT1 showing the optimization of PKMYT1 down-regulation by siRNA, in HeLa (left) and SH-SY5Y (right) cells. 'CT', control cells only exposed to the transfection agent; 'Cscr', control cells transfected with 10 pmol of a siGENOME non-targeting scrambled 'siRNAs'; 'siRNA PKMYT1', cells transfected with 5 or 10 pmol of anti-PKMYT1 siRNA. Transfection occurred for 48h and 72h. Molecular weight standard is indicated to the left. In **B.,** betalll-tubulin was also immunoprobed (lower blot). **C.** and **D:** Effects of PKMYT1 knockdown on SH-SY5Y cells metabolic rate and cell number. SH-SY5Y cells with previously PKMYT1 knockdown (10 pmol of anti-PKMYT1 siRNA, 72h: 'PKMYT1 KD'), and further replated (^{~}4.0 x 10⁴ cells per well). **C.** Metabolic rate at day 1 ('D1') and day 4 ('D4') after replating, assessed by the resazurin assay, and expressed as fold change against control cells ('CT', cells incubated with the transfection reagent only) at day 1. **D.** Cell score, per well, at 1 day after replating, using the Trypan Blue assay. N = 4 replicates. Statistically significant differences using two-way ANOVA ('*'; in **C.)** and two-tailed unpaired t test ('⁺'; in **C**. and **D**.) are presented. Statistic symbols: */⁺*P* < 0.05; ⁺⁺⁺*P* < 0.001; ⁺⁺⁺⁺*P* < 0.0001.

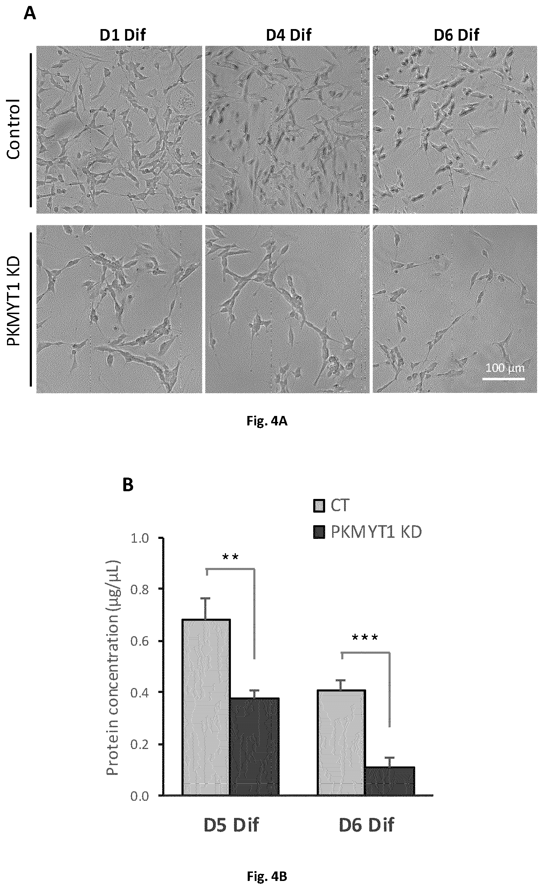

**Figure 4A** - **4C****: Effects of PKMYT1 loss-of-function on cell number and neuritogenesis during neuronal differentiation. A.** and **B.** SH-SY5Y control cells ('CT') and SH-SY5Y cells with previous PKMYT1 knockdown by siRNA for 72h ('PKMYT1 KD'), were subjected to a neuronal differentiation program: 5 days under 10 µM RA in 1 FBS % medium ('D1 Dif'-'D5 Dif') followed by 1 day under 10 ng/mL BDNF in FBS-free medium ('D6 Dif'). **A.** Phase contrast microphotographs of the cells differentiated for 1, 4 and 6 days. Bar = 100 µm. **B.** Total protein concentration of cellular lysates collected at D5 Dif and D6 Dif. N = 3 replicates. **C.** SH-SY5H cells underdoing differentiation with 10 µm RA in 1% FBS medium for 3 days ('D3 Dif CT') were simultaneously transfected with 10 pmol anti-PKMYT1 siRNA knockdown for 72 h ('D3 Dif PKMYT1 KD'). 'D0 CT', SH-SY5Y cells 24 h after plating, before initiating differentiation or PKMYT1 knockdown. Bar = 50 um. N = 4 replicates. Statistically significant differences by the two-tailed unpaired t test are presented. Statistic symbols: **P* < 0.05; ***P* < 0.01; ****P* < 0.001; *****P* = 0.0001.

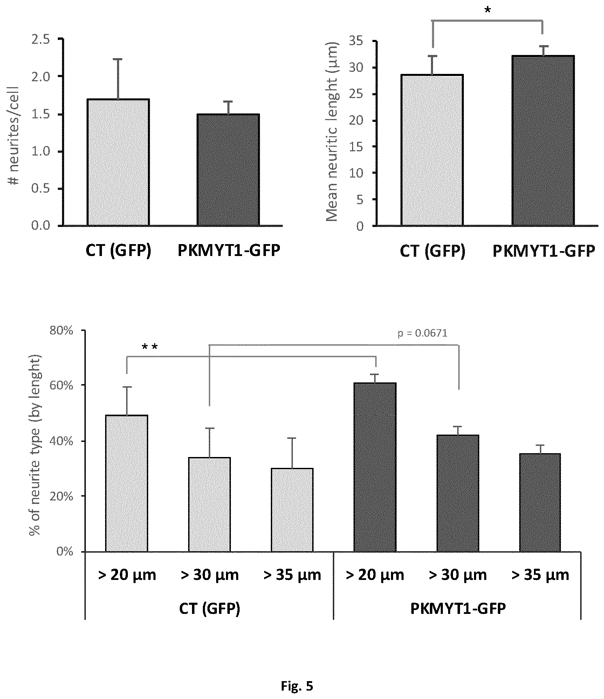

**Figure 5****: Effects of PKMYT1 gain-of-function on neuritogenesis.** SH-SY5Y cells were transfected for 2 days with 2 µg of pEGFP empty vector (CT, GFP) or pEGFP-PKMYT1 cDNA (PKMYT1-GFP), and simultaneously incubated with 10 µM RA (jn 1 FBS % medium). Fluorescence microphotographs were taken with a Zeiss Axioimage Microscope, and neuritogenesis analysed in green fluorescing cells using NeuronJ plug-in on ImageJ. N=8 images or sets of images. Statistically significant differences by the two-tailed unpaired t test are presented. Statistic symbols: **P < 0.01.

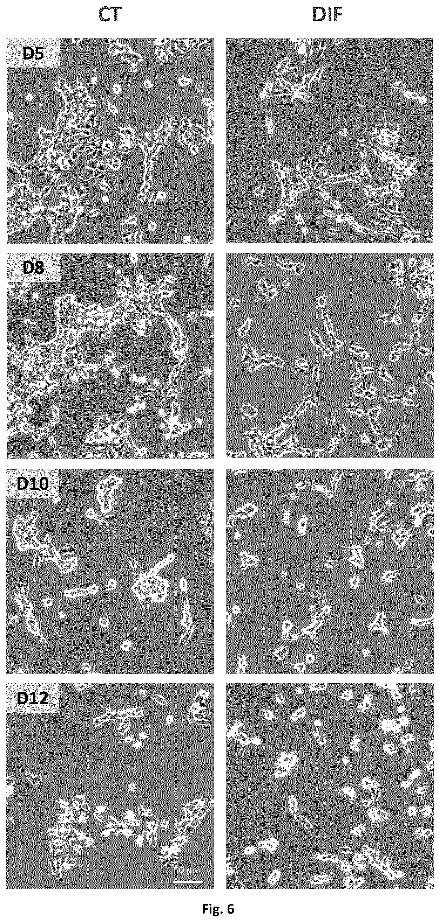

**Figure 6****: Phase contrast microphotographs of SH-SY5Y cells under proliferative (control undifferentiated, 'CT') and neuronal differentiation ('Dif') conditions.** SH-SY5Y cells were seeded at ^{~}3.0 x 10⁴/cm² and incubated with 10 µM retinoic acid in reduced serum medium for five days (D0-D5), followed by incubation for seven days (D6-D12) with 10 ng/ml brain-derived neurotrophic factor (BDNF) in serum-free medium. Control cells were grown under the same serum conditions. Bar = 50 µm.

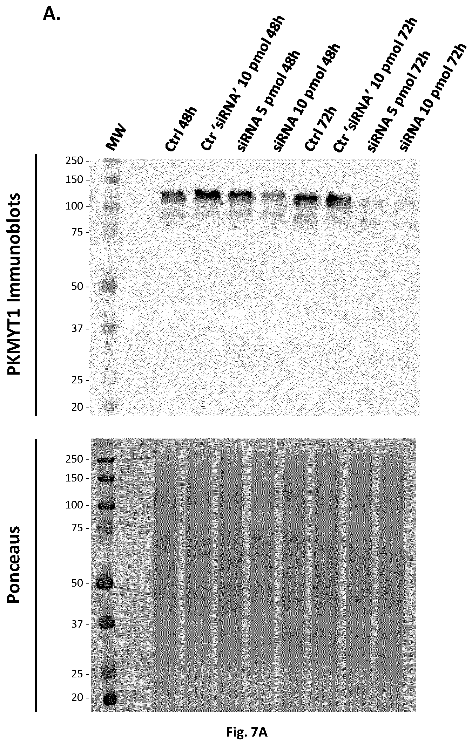

**Figure 7A** - **7B****. Optimization of PKMYT1 down-regulation by siRNA in HeLa (A) and SH-SY5Y (B) cells.** In both membranes, the loading sequence was: cells without treatment ('Ctrl'), cells transfected with 10 pmol of siGENOME non-targeting control siRNAs ('Ctrl siRNA 10 pmol'), cells transfected with 5 and 10 pmol of PKMYT1 siRNA ('siRNA X pmol'); in the first set of 4 conditions transfection occurred for 48h and in the second set for 72h. Upper membranes were incubated with the anti-PKMYT1 antibody, and lower membranes are the respective Ponceau staining. 'MW' = molecular weight standard.

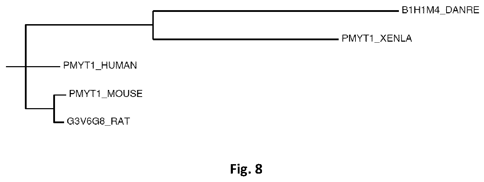

**Figure 8****: Phylogenetic tree of the human, mouse, rat, African clawed frog and zebrafish PKMYT1 amino acid sequences.** PKMYT1 phylogenetic tree using respective species specific PKMYT1 amino acid FASTA sequences, retrieved from UniProtKB. PKMYT1 sequences from rat and zebrafish (G3V6G8 and B1H1M4) are not curated.

### DETAILED DESCRIPTION

The present disclosure relates to isolated or artificial nucleotide sequences encoding the protein kinase, membrane-associated tyrosine/threonine 1 (PKMYT1), wherein the sequence encoding the protein PKMYT1 is identical to a sequence selected from a list consisting of: SEQ. ID. 1, SEQ. ID. 6, SEQ. ID. 7, for use in the treatment or regeneration of neurons following traumatic injuries on the central or peripheral nervous systems, preferably in the treatment of spinal cord injury. Furthermore, the present invention is also related to a vector comprising such sequence, a host cell comprising such vector, a protein PKMYT1, or a composition thereof, said vector, host cell, protein and composition for use in the treatment or regeneration of neurons following traumatic injuries on the central or peripheral nervous systems, preferably in the treatment of spinal cord injury.

In an embodiment, the vector used according to the present invention is a non-viral vector. Typically, the non-viral vector may be a plasmid encoding the protein PKMYT1. This plasmid can be administered directly or through a liposome, an exosome or a nanoparticle.

### Viral vectors

Viral vectors for gene delivery and/or transfer, useful in the practice of the present invention, can be constructed using methodologies well known in the art of molecular biology. Typically, viral vectors carrying transgenes are assembled from polynucleotides encoding the transgene, suitable regulatory elements and elements necessary for production of viral proteins which mediate cell transduction.

The terms "gene transfer" or "gene delivery" refer to methods or systems for reliably inserting foreign DNA into host cells. Such methods can result in transient expression of non-integrated transferred DNA, extrachromosomal replication and expression of transferred replicons (e. g. episomes), or integration of transferred genetic material into the genomic DNA of host cells.

In an embodiment, examples of viral vector include adenovirus, lentivirus, retrovirus, herpesvirus and Adeno-Associated virus (AAV) vectors.

Such recombinant viruses may be produced by techniques known in the art, such as by transfecting packaging cells or by transient transfection with helper plasmids or viruses. Typical examples of virus packaging cells include PA317 cells, PsiCRIP cells, GPenv+ cells, HEK 293 cells, etc. Detailed protocols for producing such replication-defective recombinant viruses may be found for instance in WO95/14785, WO96/22378, US5,882,877, US6,013,516, US4,861,719, US5,278,056 and WO94/19478.

### Delivery of vectors

Methods of delivery, or administration, of non-viral and viral vectors to neurons and/or astrocytes and/or oligodendrocytes and/or microglia and/or neural stem cells and/or other cells relevant for the regeneration of the tissue, include generally any method suitable for delivery vectors to said cells, directly or through cells transduction, such that at least a portion of cells of a selected cell population is transduced. The vector may be delivered to any cells of the central nervous system, cells of the peripheral nervous system, or both. Preferably, the vector is delivered to cells of the central nervous system; more preferably to cells of the spinal cord.

Additional routes of administration may also comprise local application of the vector under direct visualization, e. g., superficial cortical application, intranasal application, or other nonstereotactic application.

Preferably, the present disclosure comprises intraspinal administration.

### EXAMPLES

### Expression of PKMYT1 protein forms in neuronal-like cells

PKMYT1 expression was first screened in different cell lysates, including HeLa and MCF-7 cell lines, where PKMYT1 was described to be present/relatively abundant, according to The Human Protein Atlas. A pair of intense, conserved, bands could be found at approximately 90 and 120 kDa in all cell lysates **(****Figure 1A****,** arrows). Other fainter bands were also found near the expected molecular weight (MW) for immature nascent PKMYT1, around 54.5 kDa **(****Figure 1A****,** arrowheads).

To better understand the identified intense PKMYT1 bands of higher MW, potential PKMYT1 post-translational modifications (that are kept under denaturing conditions such as SDS-PAGE) were searched with the ELM (Eukaryotic Linear Motif) software tool (in the ExPASy portal). The ELM report for the PKMYT1 sequence (UniProt Entry Q99640) included the know globular protein kinase domains between Phe110 and Leu356 (Pkinase_Tyr) or Leu359 (Pkinase), and several post-translational modifications: nineteen modification motifs ('MOD_') were found, most of these (16) associated with phosphorylation by different kinases, in accordance with the literature data on PKMYT1 phosphorylation. For example, PKMYT1 inactivation through phosphorylation by PLK1 kinase at Ser426, Ser435, and Thr495 residues has been experimentally proved, and highly phosphorylated PKMYT1 MW bands have been observed in immunoblots when cells were about to enter in mitosis or meiosis. The other three MOD motifs retrieved that may greatly increase the molecular weight of the mature PKMYT1 protein are related to the addition of mannose and glycosaminoglycan, and SUMOylation **(****Figure 1B****,** highlights). In sum, PKMYT1 is expressed in several cell lines, including the widely used neuronal model SH-SY5Y neuroblastoma cells. Its protein forms have higher MW than first expected, most probably due to post-translational modifications, like glycosaminoglycan attachment and mannosylation.

The cellular location of PKMYT1 was also assessed in HeLa and SH-SY5Y cells, through ICC **(****Figure 1C****).** The immunofluorescence microphotographs of both cell types showed PKMYT1 distributed in the cells' nuclei (arrows), both in the nucleoli and nucleoplasm and in the cells' cytoplasm (arrowheads), in accordance with PKMYT1 nuclear functions and the protein's subcellular localization described in The Human Protein Atlas.

### PKMYT1 protein profiles with cellular proliferation and neuronal differentiation

The main function attributed to PKMYT1 is as a cell cycle regulator⁶. This function has been assessed in animal oocytes and zygotes, Sf9 insect cells, HeLa cells and other cancer cell lines (such as non-small lung cancer cells), but not in neuroblastoma cells, which are similar to neural precursor cells (NPCs) when non-differentiated. PKMYT1 protein expression profile was thus monitored in proliferative SH-SY5Y neuroblastoma cells, grown until confluency, achieved between days 5 and 6 (D5-D6) **(****Figure 2A****).** PKMYT1 protein levels increased linearly over time in culture until D5, a time point where the protein reached an expression *plateau,* followed by a slight decrease from D6 to D7.

The following analysis aimed to monitor the changes in PKMYT1 protein expression during the neuronal differentiation process, to assess possible relations between PKMYT1 and the induction of a neuronal-like phenotype **(****Figure 2B****).** For this, SH-SY5Y neuroblastoma cells were committed to neuronal differentiation for 5 days (D0-D5) with 10 µM retinoic acid (RA) in a low serum media (1% FBS), and further fully neuronally differentiated with 10 ng/mL Brain-derived neurotrophic factor (BDNF) in serum-free media, from D5 to D12. PKMYT1 protein levels were monitored and, when compared to control undifferentiated cells under the same serum conditions, were observed to be tendentially lower in differentiated cells during most of the neuronal differentiation time course, until D8 (when its levels are significantly lower). However, at D10, PKMYT1 levels greatly decreased in control cells, and this protein's levels were significantly higher in the differentiated population. Microphotographs of the undifferentiated and differentiated populations **(****Figure 2B****,** right; **Figure 6****)** show that: a) there was an increased cell death from D8 to D10, much more pronounced for control cells, with both populations reaching a similar number of cells at that time point; b) differentiating cells presented longer and thinner neuritic projections at D10.

### Effects of PKMYT1 knockdown on the number and cellular metabolism of neuroblastoma cells

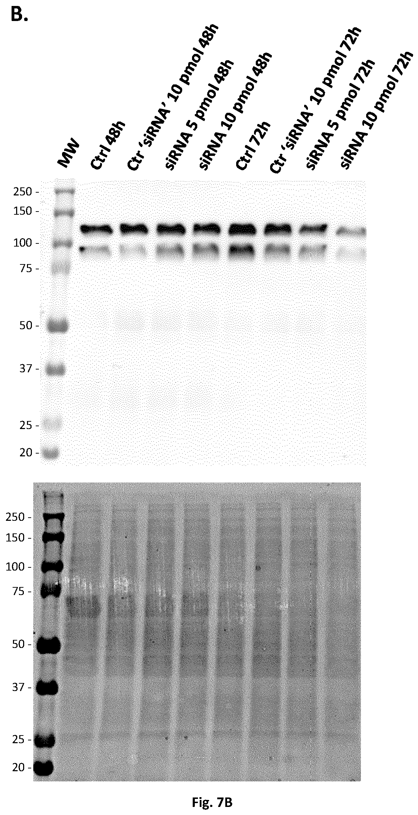

To characterize PKMYT1's potential neuronal functions, PKMYT1 loss-of-function experiments were carried out. Firstly, the efficacy of PKMYT1 down-regulation using small interfering RNA (siRNA) transfection, was tested in HeLa and SH-SY5Y cells. Decreases in PKMYT1 levels were induced by 10 pmol of PKMYT1 siRNA, as earlier as 48 h post-transfection in HeLa cells, but more effectively at 72 h, (when only 5 pmol siRNA were also effective), and at 72 h in SH-SY5Y cells **(****Figures 3A** **and B;** full blots and respective Ponceau staining in **Figure 7****).**

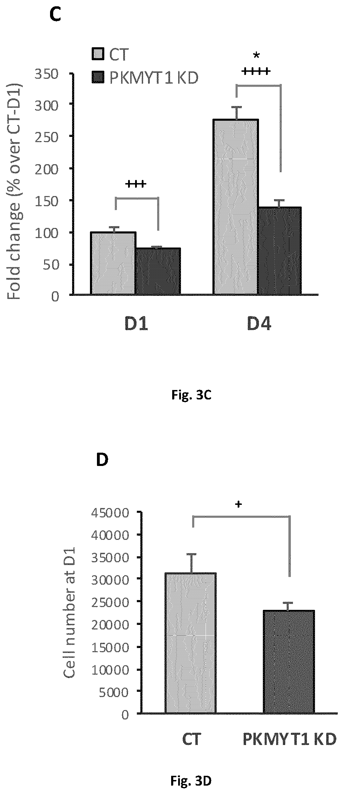

Following, a cell proliferation assay was performed in SH-SY5Y cells previously knockdown for PKMYT1. Cells were first transfected with 10 pmol of the anti-PKMYT1 siRNA for 72 h and further replated (^{~}4.0 x 10⁴ cells per well) and grown for 4 days. The metabolic rate of these cells was monitored on day 1 and day 4, through the non-toxic resazurin assay; the number of cells per plate was scored on day 1 with Trypan Blue **(****Figures 3C and D****).** Upon 1 day after replating, PKMYT1 down-regulated cells ('PKMYT1 KD') presented only 74.0±3.3 % of the metabolism of respective control cells on day 1 ('CT' at 'D1', taken as 100%). Accordingly, the number of PKMYT1 KD cells was also 73.8±6.0 % of control cells at D1 **(****Figure 3C****).** Following 4 days ('D4'), the metabolism of the control cells increased to 276±19.3 %, while the metabolism of PKMYT1 KD grew only to 138.8±12.2%, which represented 50.3±4.4% of the metabolism of control cells at D4 **(****Figure 3C****).** Hence, PKMYT1 knockdown resulted in a reduction in the number of SH-SY5Y proliferative, neuronal precursor-like, cells.

### Impairment of neuronal differentiation in PKMYT1 knockdown cells

The protein profile of PKMYT1 during neuronal differentiation revealed a potential functional relation between PKMYT1 and neuritic elongation **(****Figure 2B****).** Further, the levels of βIII-tubulin (the neuronal tubulin isoform) appear to decrease with the knockdown of PKMYT1 in undifferentiated cells **(****Figure 3B****).** Hence, a potential function for PKMYT1 in neuronal differentiation was further tested using SH-SY5Y cells previously knockdown for the protein (as in the metabolic assay of **Figure 3C****).** After 72 h of anti-PKMYT1 siRNA transfection, control and PKMYT1 down-regulated cells were plated at ~3.5 x 10⁴/cm² in complete medium (10% FBS) and, after 24 h, were subjected to differentiation assay with RA in low serum conditions and BDNF in serum-free conditions, as above. Phase contrast microscopy pictures were taken at D1 and D4 after RA addition, and at D6, 1 day after BDNF addition **(****Figure 4A****).** Again, a major negative effect on cell number was observed: although the same number of cells was initially seeded in both conditions, fewer cells could be seen in the PKMYT1 KD images, as soon as 1 day after RA addition. Cells were further collected following D5 and D6 of differentiation, for analysis of total protein concentration **(****Figure 4B****).** In accordance with the lower cell number observed in the microphotographs, lower total protein concentrations were obtained in the PKMYT1 knockdown differentiating population, relatively to the control differentiating one. This difference is accentuated after treatment with BDNF in serum-free media ('D6 Dif') when the protein concentration of the PKMYT KD population is nearly only ~25% of the respective control cells' value.

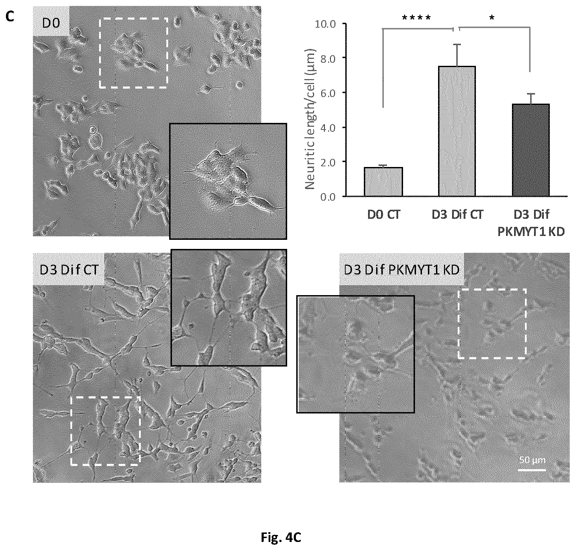

Given that PKMYT1 knockdown before replating results in a severe decrease in the number of cells, the effects of PKMYT1 loss-of-function on SH-SY5Y cells neuritogenesis were assessed by performing PKMYT1 knockdown by siRNA alongside RA-induced differentiation for 3 days. Phase contrast microphotographs were taken at D0 (undifferentiated cells) and at D3 of neuronal differentiation, for both control and siRNA PKMYT1 KD conditions, and the neuritic network was evaluated using the NeuroRead macro for ImageJ, which retrieves the cell body number and the total neuritic length per image **(****Figure 4C****).** As expected, from D0 to D3, control cells responded to RA treatment and shifted from a more polygonal symmetric undifferentiated morphology to a more elongated one with a higher number of cellular projections. Quantitatively, the neuritic length per cell significantly increased 5-fold (from 1.62±0.15 to 7.48±1.30 µm). When differentiating cells were simultaneously subjected to a continuous PKMYT1 knockdown, the cellular morphology was less polarized and the average total neuritic length per cell only increased to 5.32±0.59 µm (p<0.05 vs 7.48±1.30 µm, the control cells values).

### Increased PKMYT1 is associated with neuritic elongation and neuronal regeneration

All previous assays involving neuronal differentiation point towards a PKMYT1 role in neuritic elongation. To prove the PKMYT1 role in neuritic elongation, a gain-in-function experiment was performed, in which the PKMYT1 cDNA of isoform 1 (NM_004203.5) (SEQ. ID. 6), with the nucleotide coding sequence (CDS) CCDS ID: CCDS10486.1 (NCBI database) (SEQ. ID. 7) was obtained from SH-SY5Y cells RNA, and cloned in fusion with the EGFP cDNA (SEQ. ID. 12, an embodiment outside the subject-matter of the claims but useful for understanding the disclosure ), obtaining the EGFP-PKMYT1 fusion cDNA (SEQ. ID. 1) inside a pEGFP-expressing vector.

In an embodiment, the resulting pEGFP-PKMYT1 plasmid was transfected for 48h in cells being simultaneously differentiated with RA **(****Figure 5****).** An analysis using NeuronJ v1.4.3 plugin for ImageJ compared microphotographs from cells transfected with an empty pEGFP and cells transfected with pEGFP-PKMYT1. Confirming the surprising neuritogenic PKMYT1 role, the mean neuritic length was significantly higher in the PKMYT1 up-regulated cells (32.07±1.79) when compared to control (28.46±3.63) (p-value=0.027) **(****Figure 5****;** top right). Further, neurites from PKMYT1 up-regulated cells were comparatively longer since 61% of them were at least 20 µm long, 42% were at least 30 µm long, and 35% were at least 35 µm long, contrasting with the control cells (49%, 34% and 30%, respectively) **(****Figure 5****;** bottom). The ratios between neurites and number of cells were similar between groups **(****Figure 5****;** top left).

All the assays above have been performed *in vitro,* in cell culture models. To assess a possible relation between PKMYT1 and neuronal regeneration, its expression was monitored in our dataset of potential Regeneration-associated genes (RAGs). To obtain this dataset, we have performed RNA sequencing of Dorsal Root Ganglion (DRG) neurons collected 24 h after two lesion paradigms: a) a sciatic nerve transection, which naturally regenerates (peripheral nerve injury, PNI); and b) a spinal cord transection, which does not regenerate (spinal cord injury, SCI). RNA from DRGs of sham-operated control animals was sequenced in parallel. Surprisingly, PKMYT1 was found to be highly up-regulated in regenerating DRG neurons (PNI condition), when compared to DRG neurons of sham-operated control animals, presenting a Log₂FC of 2.85 (p = 0.017), what corresponds a 7.21 increase over basal conditions. Contrarily, this gene was not altered after a non-regenerative SCI, at least at this time point **(Table 1,** shaded rows).

**Table 1. Up-regulation of PKMYT1 mRNA levels in neuronal regenerative conditions. Shaded rows: data from our transcriptomic study on DRG neurons at 1 day post-injury (dpi), following a regenerating PNI or a non-regenerating SCI. Log₂FC, logarithm of the fold change (FC) of PKMYT1 experimental mRNA levels over its mRNA levels in sham-operated (non-neural lesioned) control animals. Unshaded rows: comparative in silico study of transcriptomic data from various spinal cord naturally regenerating animals.**

| **Species** | **Lesion Model** | **Cell/tissue collected** | **Time points** | **Log2(FC)** | **FC** | **P-value** | **Source** |
|---|---|---|---|---|---|---|---|
| *Rattus norvegicus* | Sciatic Nerve Injury (PNI) | Dorsal Root Ganglia neurons | 1 dpi | 2.85 | 7.21 | 0.017 | Our transcriptomic study on DRG neurons response to regenerative PNI vs non-regenerative SCI |
| | Spinal Cord Injury (SCI) | | | - | - | ns | |
| *Danio rerio* | Spinal Cord Injury | Neurons of the medial longitudinal fascicle nucleus (NMLF) | 0.5 dpi | 0.88 | 1.84 | 0.91 | Ma et al., 2014¹² |
| | | | 11 dpi | 6.57 | 95.00 | 0.45 | |
| *Petromyzo n marinus* | Spinal Cord Injury | Spinal cord tissue (1 cm surrounding transection site) | 1 dpi | 1.06 | 2.08 | 0.59 | Herman et al., 2018¹³ |
| | | | 3 dpi | 0.55 | 1.46 | 0.92 | |
| | | | 7 dpi | 0.66 | 1.58 | 0.90 | |
| *Xenopus laevis* | Spinal Cord Injury | Spinal cord segment caudal to the transection site (7^{th}-8^{th} TV) | 1 dpi | 0.33 | 1.25 | 0.03 | Lee-Liu et al., 2014¹⁴ |
| | | | 2 dpi | 0.24 | 1.18 | 0.22 | |
| | | | 6 dpi | -0.42 | 0.75 | 0.00 | |

A parallel *in silico* analysis was performed by us in non-mammalian vertebrates known to spontaneously and fully regenerate a spinal cord following injury (e.g. the tadpoles of *Xenopus* genus). A search for studies with transcriptomic datasets of SCI- vs sham-operated animals, using spinal cord regenerative animals, was conducted throughout transcriptomic databases and *PubMed.* Six studies retrieved were included for analysis due to being complete (having a control group), not testing drugs or any therapy, and having at least N=3 **(Table 2),** three studies were excluded due to: lack of data from control groups; shortage of replicates; assessment of drugs on recovery; or unpublished data.

**Table 2. Transcriptomic studies in spinal cord regenerating vertebrate species.**

| **Organism** | **Analysis Type** | **Date** | **Study** | **Ref** |
|---|---|---|---|---|
| *Danio rerio* | Microarray | 2011 | Guo et al., 2011 | 15 |
| *Danio rerio* | Microarray | 2014 | Hui et al., 2014 | 16 |
| *Danio rerio* | Microarray | 2014 | Ma et al., 2014 | 12 |
| *Petromyzon marinus* | RNA-Sequencing | 2018 | Herman et al., 2018 | 13 |
| *Xenopus laevis* | RNA-Sequencing | 2014 | Lee-Liu et al., 2014 | 14 |

The transcriptomic data of the lesioned spinal cord tissue or affected neurons responses of the five studies was processed (please see Materials and Methods). PKMYT1 transcripts appeared deregulated in four of these five studies' datasets. PKMYT1 expression levels were tendentially up-regulated (positive log₂ FCs) in three of the four studies, at five (out of six) timepoints (days post-injury, 'dpi'): in *Danio rerio* at 0.5 and 11 dpi¹², in *Petromyzon marinus* at 1, 3 and 7 dpi¹³ and in *Xenopus laevis* at 1 and 2 dpi¹⁴, although only statistically significant in *X. laevis* at 1 dpi (log₂ FC = 0.33, p = 0.0322) **(Table 1,** unshaded rows). To discard critical homology differences between the PKMYT1 enzymes of these animals and mammalian ones, an alignment between human, mouse, rat, African clawed frog, and zebrafish PKMYT1 amino acid sequences was performed using Clustal Omega (the *Petromyzon marinus* UniProtKB Entry was not available in this database). This analysis revealed that despite some phylogenetic distance and protein identity difference **(****Figure 8** **and Table 3,** respectively) between the analysed mammalian vs regenerative vertebrate species, all of them maintain the strongly conserved amino acids of the active site of the PKMYT1's kinase domain (Asp123, Asn128 and Asp141), supporting its involvement in PKMYT1 neural regeneration functions.

**Table 3. Identity percentage matrix of PKMYT1 in different vertebrate animal species. Comparisons between the human, mouse, rat, African clawed frog, and zebrafish PKMYT1 amino acid sequences. In the second column, UniProt Entry is specified before each species denomination. Tree and matrix created with Clustal2.1. 'DANRE' = Danio rerio (zebrafish); 'XENLA' = Xenopus laevis (African clawed frog); 'HUMAN' = Homo sapiens; 'MOUSE' = Mus musculus; 'RAT' = Rattus norvergicus.**

| | | **DANRE** | **XENLA** | **HUMAN** | **MOUSE** | **RAT** |
|---|---|---|---|---|---|---|
| **1** | **B1H1M4_DANRE** | **100.00** | 39.53 | 42.86 | 42.23 | 42.02 |
| **2** | **Q91618_XENLA** | 39.53 | **100.00** | 51.12 | 50.31 | 50.72 |
| **3** | **Q99640_HUMAN** | 42.86 | 51.12 | **100.00** | 89.39 | 89.80 |
| **4** | **Q9ESG9_MOUSE** | 42.23 | 50.31 | 89.39 | **100.00** | 96.94 |
| **5** | **G3V6G8_RAT** | 42.02 | 50.72 | 89.80 | 96.94 | **100.00** |

In sum, PKMYT1 is induced in regenerating neurons (pending on the animal and analysed time points), following traumatic injuries inflicted on the central or peripheral nervous systems of various animal models.

Spinal cord injury (SCI), like several other central neuropathologies, has no effective treatment and is highly deleterious to the patients, caregivers and the Healthcare system. New effective therapeutic strategies for SCI are mandatory, to reduce its high detrimental impact and return quality of life to SCI patients³. We have been focusing our research on identifying novel neuroregeneration-associated genes (RAGs), whose modulation in genetic and molecular therapies promotes the regeneration of neurons following SCI or other central neuropathologies. Strategies for the identification of such RAGs entail performing and analyzing comparative transcriptomic studies on the gene expression responses of neurons in regenerative versus non-regenerative conditions.

One of the genes that we have experimentally observed to be significantly up-regulated in rat DRG neurons upon a regenerative lesion (PNI), but not when under a non-regenerative one (SCI), was PKMYT1 **(Table 1).** Our *in silico* analyses also found this gene to be significantly up-regulated in the spinal cord regenerating *Xenopus laevis,* 1 day after a SCI in the spinal cord segment caudal to the transection site **(Table 1),** and in the injured spinal cord of *Mus musculus* knockout for EphA4, an SCI mouse model with increased motor recovery scores.

PKMYT1 is known to play a key role in cell cycle arrest during G2/M in response to DNA damage⁷, which may be important in regeneration to increase the asymmetric division of NPCs, giving rise to more newly-born neurons to replace dead cells. But PKMYT1 may be also involved in other major cellular processes crucial for neuronal regeneration, including apoptosis events required to clean the cellular debris, and neuritogenesis, particularly important the regrowth of axons following SCI and PNI.

Regarding the role of PKMYT1 in neural proliferation and cell survival, the present disclosure comprises a series of assays using proliferative neuroblastoma cells, a widely used model of NPCs. PKMYT1 protein levels were observed to steadily increase during the SH-SY5Y population growth, accompanying the usual log proliferative phase, in accordance with the main role of this protein on cell division quality control **(****Figure 2A****).** Further loss-of-function experiments revealed that PKMYT1 down-regulation leads to a reduction in SH-SY5Y cell number (and concomitantly decreased metabolism of the cell population) **(****Figure 3C-D****).** A reduction in the number of PKMYT1 knockdown cells (and related total protein content) was also visible upon RA exposure in reduced serum conditions during a neuronal differentiation assay **(****Figure 4A and B****).** A similar effect had already been reported in KYSE450/70 esophageal squamous carcinoma cells, and in A549 and H1299 cells (non-small cell lung carcinoma cells). The reduction in cell number upon PKMYT1 down-regulation is most likely due to a dysregulated cell cycle, which accumulates DNA mutations leading to genetic instability and apoptosis. This is particularly true for cancer-derived cell lines, such as SH-SY5Y neuroblastoma cells. Indeed, since cancer cells commonly have a faulty G1 checkpoint mechanism caused by defective p53/Rb pathways, making the G2 phase essential in cancer cells' survival, PKMYT1 and WEE1⁶ are both frequently overexpressed in various cancer types to maintain cell genomic stability under rapid cell proliferation¹¹. On the other hand, in conditions of down-regulation of the WEE kinase family, most cancer cells become more unstable and predisposed to apoptosis, also becoming more sensitive to applied treatments. Down-regulation of WEE kinase family members results in premature activation of CDK1, a protein that signals cell cycle progression to mitosis, initiating mitotic events in the S phase and ending in cell death/embryonic lethality¹¹. Indeed, PKMYT1 (and WEE1) usually inhibit the activity of CDK1¹¹ as early as the G1/S transition, to prevent entering mitosis during DNA replication and subsequent apoptosis. Accordingly, in *D. melanogaster,* mutated PKMYT1 causes cell-cycle defects, ectopic apoptosis and abnormal responses to ionizing radiation, due to the disruption of CDK1 regulatory functions. In hepatic steatosis, EGFR deficiency and reduced PKMYT1 and WEE1 expression in hepatocytes that attempt to regenerate, lead to mitotic impairments and subsequent defective regeneration of fat livers. Hence, the observed reduced number of SHSY-5Y cells upon PKMYT1 down-regulation is most probably due to CDK1 abnormal early activation and associated commitment to apoptosis.

The present disclosure describes the role of PKMYT1 on neuronal differentiation, particularly on neuritogenesis. A neuronal differentiation time-course, monitoring the levels of PKMYT1 throughout the neuronal differentiation process **(****Figure 2B****),** showed that PKMYT1 protein levels tend to decrease during the first two-thirds of the *in vitro* RA+BDNF differentiation program, when cells start emitting more new cellular projections of short length. This tendential decrease is potentially due to a general lower cell proliferation rate induced by RA in low serum media (D1-D5 Dif), and total proliferation arrest induced by cells exposure to BDNF in serum-free media (D6-D8). However, at D10 of differentiation, when the division-arrested differentiating cells that survived total serum-withdrawn appear to undergo a neuritic elongation phase, the opposite occurs and PKMYT1 levels are higher in differentiated cells. Indeed, PhC microphotographs of neuronally differentiated cells show that, from D8 to D10, the number of cells decrease and the cellular neurites are generally longer. This shows that PKMYT1 is involved in neuritic elongation, and again potentially in cell survival. Accordingly, PKMYT1 levels in control conditions at D10 are quite lower due to high cell death from D8 to D10 **(****Figure 2B** graph). Strengthening a role for PKMYT1 in neuritogenesis, PKMYT1 knockdown assays have resulted in decreased levels of βlll-tubulin, a cytoskeleton protein that correlates with neuronal differentiation **(****Figure 3B****),** and in decreased neuritic outgrowth even upon the first 3 days of the neuronal differentiation program **(****Figure 4C****).** In contrast, overexpression of the PKMYT1 protein, via transfection of its gene inside pEGFP-PKMYT1, at this early differentiation period resulted in increased neuritic elongation **(****Figure 5****).** Of note, these two processes (generation of new neuritic projections and their subsequent elongation) appear to be competitive (our unpublished observations).

In sum, PKMYT1 is a neuritic elongation-promoting molecule of high relevance for axonal regeneration following SCI. The *in vitro* neuritogenic data of the present disclosure corroborate the observations of upregulated PKMYT1 transcriptomic levels in regenerating DRG neurons upon a PNI and in neural tissue of naturally regenerating animals **(****Figure 5****),** where augmented PKMYT1 promote regrowth of damaged axons. This effect is clearly mediated by PKMYT1 in its protein form, since its cDNA and mRNA encoding acid nucleic molecules have no other biological function than the one of coding the PKMYT1 protein. The GFP cDNA, or protein encoded by that cDNA and expressed in the pEGFP transfected cells, widely used in research, are also not responsible for the neuritogenic effects. Mechanistically, PKMYT1 positive effects on neuronal differentiation may be associated with an inhibition of proliferation. These events are connected, and the arrest of cell division is important for the cellular commitment to differentiation, which was already proved in SH-SY5Y cells differentiation. In the present disclosure, neuronal differentiation was achieved by treating cells with retinoic acid (RA), while decreasing serum supplementation, which induces both cell cycle arrest and early (immature) neuronal differentiation. Moreover, and as stated above, CDK1 knockdown in hESCs/hiPSCs stem cells down-regulates pluripotency markers and up-regulates differentiation ones. In opposite, CDK1 promotes pluripotency events via CDK1-mediated phosphorylation of PDK1, subsequent activation of PI3K/Akt and its effectors ERK and GSK3β, with the activation of the CDK1-PDK1-PI3K/Akt kinase signaling cascade favoring self-renewal and pluripotency acquisition. The interaction of PKMYT1 with the CDK1-cyclin A complex is a potential mechanism to couple mitosis exit and cell differentiation. In terms of other potential pathways by which this protein exerts its NPC/neuronal-related functions, PKMYT1 may be a downstream target of the Notch signaling pathway, a known player in nervous system development and neuronal differentiation. PKMYT1 neuronal functions may be also mediated by BRSK 1 and 2 (alias SAD-A and -B, respectively), that have been implied in synaptic plasticity and described to phosphorylate and negatively regulate WEE1 activity to promote differentiation of polarized neurons. Contrarily, WEE1 persistent expression interferes with neuron polarization and axon formation. The increase in PKMYT1 levels at a later differentiation time point **(****Figure 2B****,** D10) of high neuritic elongation and potential synapse formation, may occur to help the promotion of neuritic elongation and to compensate for the BRSK 1-2-mediated down-regulation of WEE1 activity during neuronal differentiation. In this way, PKMYT1 positive effects in neuronal differentiation and neuritogenesis appear to differ from those of the other WEE proteins of its family.

In synthesis, the present disclosure describes the use of PKMYT1 in regenerative medicine, namely in neuronal cellular processes relevant for regeneration, namely in the control of cell number and promotion of neuritic elongation. In post-mitotic neurons, the phosphorylating activity of PKMYT1 may exert a function in regulating signaling cascades that may act towards neuronal survival, differentiation, and even synapse formation. PKMYT1 expression levels may vary during nervous system development, adulthood and adult neuroregeneration, according to the need of cellular repopulation versus the need of differentiation. A well-regulated and temporally PKMYT1 expression to meet these needs will most likely promote a careful and balanced organization of the complex architecture of neuronal differentiation and regeneration processes, without generating side-effects. The present disclosure shows that PKMYT1is a suitable target of new effective therapies for SCI and other central and peripheral neuropathologies.

### MATERIALS AND METHODS

### Culture and maintenance of SH-SY5Y and HeLa cell lines

Human neuroblastoma SH-SY5Y cells (ATCC^{®} CRL-2266^{™}) were cultured in a 1:1 mixture of Minimum Essential Medium and Ham's F12 containing 2 mM L-glutamine (Gibco), and HeLa cells (ATCC^{®} CRM-CCL-2^{™}) were cultured in Dulbecco's Modified Eagle Medium (Gibco). Both media were supplemented with 10% heat-inactivated Fetal Bovine Serum (FBS), and 1% antibiotic/antimycotic solution (1 000 units/mL penicillin; 10000 µg/mL streptomycin and 25 µg/mL Amphotericin B) (Gibco, Invitrogen, Life Technologies). Lysates of MCF-7 cells (ATCC^{®} HTB-22^{™}), an epithelial cell line isolated from the breast tissue of a patient with metastatic adenocarcinoma, were a kind gift from the group of Dr Luisa Helguero.

### SH-SYSY proliferation and neuronal differentiation assays

To assess PKMYT1 expression throughout proliferation, SH-SY5Y cells were seeded at ^{~}4.0 x 10⁴/cm² in complete medium and grown for 7 days. Lysates were collected in 1% SDS every 24h. For neuronal differentiation assays, SH-SY5Y cells - either control cells and/or cells previously transfected for 72h with anti-PKMYT1 siRNA - were seeded at ^{~}3.0 x 10⁴/cm² (time course assay of endogenous PKMYT1 levels during neuronal differentiation) or at ^{~}3.5 x 10⁴/cm² (PKMYT1 knockdown in differentiation assay). Upon 24 h post-seeding, the culture medium was changed to a differentiation medium with 10 µM all-*trans* retinoic acid (RA; Sigma-Aldrich) and 1% FBS. Upon 5 days in RA-containing medium, cells were further differentiated in serum-free medium with 10 ng/mL brain-derived neurotrophic factor (BDNF; Sigma-Aldrich) for another 7 days. Throughout these 12 days of differentiation, the medium was changed every 2-3 days. Whenever the medium was changed and 24 h after the addition of RA or BDNF, cell lysates were collected in 1% sodium dodecyl sulphate (SDS) and phase contrast (PhC) microphotographs were acquired in an Olympus IX-81 widefield epifluorescence inverted microscope.

### PKMYT1 knockdown by siRNA

Anti-PKMYT1 siRNA transfections were performed 24 h after cell seeding, using 5-10 pmol of anti-PKMYT1 Silencer^{®} Pre-designed siRNA (ID 118297, Life Technologies; sense: GUGGCAUGCAACAUGGAGCtt; antisense: GCUCCAUGUUGCAUGCCACtt), and Lipofectamine^{™} RNAiMAX (ThermoFisher) as transfection reagent. Control groups were composed of non-transfected cells, cells treated with the transfection reagent alone or transfected with 10 pmol of siGENOME Non-Targeting Control siRNA #3 (D001210-03-05; Dharmacon). Cells were transfected for 48 or 72 h, as indicated for each assay.

### Cellular metabolism and cell score in PKMYT1 knockdown cells

SH-SY5Y cells were first transfected with 10 pmol of the anti-PKMYT1 siRNA for 72 h. PKMYT1 down-regulated cells were detached, re-plated at ^{~}2.0 x 10⁴/cm² and grown for 4 days in complete medium. Control cells, plated in parallel, were only incubated with Lipofectamine^{™} RNAiMAX (ThermoFisher); this treatment resulted in similar basal PKMYT1 levels as transfection with the control siRNA (siGENOME Non-Targeting Control siRNA #3; data not shown). The relative cellular metabolic rate was assessed on day 1 and day 4 after PKMYT1 knockdown for 72 h and replating. Cells were incubated for 4 h in complete medium with 10% resazurin solution (0.1 mg/mL resazurin salt in PBS, Sigma-Aldrich). The resazurin reduction to resorufin was then measured through spectrophotometry at 570 and 600 nm (Infinite 200 PRO, Tecan). The ratio between optical densities (OD 570/OD 600) was used to infer the relative cellular metabolic rate.

### pEGFP-PKMYT1 cDNA cloning and gain-in-function differentiation assay

Total RNA from SH-SY5Y was extracted with NZyol (NZYTech), and converted to cDNA using the RevertAid Reverse Transcriptase (ThermoFisher), Oligo-dT primers (Eurofins), dNTPs (NZYTech), reaction buffer (NZYTech), and RiboLock RNAse (ThermoFisher). Primers targeting the PKMYT1 coding sequence (gene ID 9088, NP_004194.3) were designed to include restriction enzymes recognition sites (PKMYT_Hindlll_FW: 5'-AATAAGCTTCCATGCTAGAACGGCCTCCT-3'; PKMYT_BamHI_RV: 5'-ATAGGATCCTCAGGTTGGGTCTAGGGT-3'). PKMYT1 was amplified with Platinum SuperFi II MasterMix (ThermoFisher) according to the manufacturer's instructions. PKMYT1 and pEGFP-C1 (Takara Clontech) were digested with *Hind*III and *Bam*HI using the double digestion protocol of the Anza restriction enzyme cloning system (ThermoFisher). Digests were purified with the NZYGelpure kit (NZYTech) before ligation with the Anza T4 DNA ligase MasterMix (ThermoFisher) in a 3:1 insert:plasmid molar ratio, which was followed by transformation of *E. coli* NZYalpha cells. Colonies were selected with kanamycin (50 µg/mL) and transformants harbouring the desired plasmid (pEGFP-PKMYT1) were identified by colony-PCR screening with NZYTaq II DNA polymerase (NZYTech). The plasmid pEGFP-PKMYT1 was further amplified and purified by midi-preparation (NucleoBond Xtra Midi kit, MACHEREY-NAGEL).

**Table 4 - The following table describes the list of sequences described in the present application, for which an accession number is not available.**

| **Seq. ID** | **Sequence name** | **Organism name** | **Molecule type** | **Qualifier Molecule Type** | **Residues** |
|---|---|---|---|---|---|
| Seq. ID. 12* | EGFP cDNA | Synthetic construct | DNA | Other DNA | ATGGTGAGCAAGGGCGAGGAGCTGTTCACCGGGGTG GTGCCCATCCTGGTCGAGCTGGACGGCGACGTAAAC GGCCACAAGTTCAGCGTGTCCGGCGAGGGCGAGGGC GATGCCACCTACGGCAAGCTGACCCTGAAGTTCATCT GCACCACCGGCAAGCTGCCCGTGCCCTGGCCCACCCT CGTGACCACCCTGACCTACGGCGTGCAGTGCTTCAGC CGCTACCCCGACCACATGAAGCAGCACGACTTCTTCA AGTCCGCCATGCCCGAAGGCTACGTCCAGGAGCGCA CCATCTTCTTCAAGGACGACGGCAACTACAAGACCCG CGCCGAGGTGAAGTTCGAGGGCGACACCCTGGTGAA CCGCATCGAGCTGAAGGGCATCGACTTCAAGGAGGA CGGCAACATCCTGGGGCACAAGCTGGAGTACAACTA CAACAGCCACAACGTCTATATCATGGCCGACAAGCAG AAGAACGGCATCAAGGTGAACTTCAAGATCCGCCAC AACATCGAGGACGGCAGCGTGCAGCTCGCCGACCAC TACCAGCAGAACACCCCCATCGGCGACGGCCCCGTGC TGCTGCCCGACAACCACTACCTGAGCACCCAGTCCGC CCTGAGCAAAGACCCCAACGAGAAGCGCGATCACAT GGTCCTGCTGGAGTTCGTGACCGCCGCCGGGATCACT CTCGGCATGGACGAGCTGTACAAGTCCGGACTCAGAT CTCGAGCTCAAGCTTCC |
| Seq. ID. 1 | EGFP-PKMYT1 fusion cDNA; the PKMYT1 cDNA is underlined | Synthetic construct | DNA | Other DNA | ATGGTGAGCAAGGGCGAGGAGCTGTTCACCGGGGTG GTGCCCATCCTGGTCGAGCTGGACGGCGACGTAAAC GGCCACAAGTTCAGCGTGTCCGGCGAGGGCGAGGGC GATGCCACCTACGGCAAGCTGACCCTGAAGTTCATCT GCACCACCGGCAAGCTGCCCGTGCCCTGGCCCACCCT CGTGACCACCCTGACCTACGGCGTGCAGTGCTTCAGC CGCTACCCCGACCACATGAAGCAGCACGACTTCTTCA AGTCCGCCATGCCCGAAGGCTACGTCCAGGAGCGCA CCATCTTCTTCAAGGACGACGGCAACTACAAGACCCG CGCCGAGGTGAAGTTCGAGGGCGACACCCTGGTGAA CCGCATCGAGCTGAAGGGCATCGACTTCAAGGAGGA CGGCAACATCCTGGGGCACAAGCTGGAGTACAACTA CAACAGCCACAACGTCTATATCATGGCCGACAAGCAG AAGAACGGCATCAAGGTGAACTTCAAGATCCGCCAC AACATCGAGGACGGCAGCGTGCAGCTCGCCGACCAC TACCAGCAGAACACCCCCATCGGCGACGGCCCCGTGC TGCTGCCCGACAACCACTACCTGAGCACCCAGTCCGC CCTGAGCAAAGACCCCAACGAGAAGCGCGATCACAT GGTCCTGCTGGAGTTCGTGACCGCCGCCGGGATCACT CTCGGCATGGACGAGCTGTACAAGTCCGGACTCAGAT CTCGAGCTCAAGCTTCCATGCTAGAACGGCCTCCTGC ACTGGCCATGCCCATGCCCACGGAGGGCACCCCGCCA CCTCTGAGTGGCACCCCCATCCCAGTCCCAGCCTACTT CCGCCACGCAGAACCTGGATTCTCCCTCAAGAGGCCC AGGGGGCTCAGCCGGAGCCTCCCACCTCCGCCCCCTG CCAAGGGCAGCATTCCCATCAGCCGCCTCTTCCCTCCT CGGACCCCAGGCTGGCACCAGCTGCAGCCCCGGCGG GTGTCATTCCGGGGCGAGGCCTCAGAGACTCTGCAG AGCCCTGGGTATGACCCAAGCCGGCCAGAGTCCTTCT TCCAGCAGAGCTTCCAGAGGCTCAGCCGCCTGGGCCA TGGCTCCTACGGAGAGGTCTTCAAGGTGCGCTCCAAG GAGGACGGCCGGCTCTATGCGGTAAAGCGTTCCATG |
| | | | | | TCACCATTCCGGGGCCCCAAGGACCGGGCCCGCAAG TTGGCCGAGGTGGGCAGCCACGAGAAGGTGGGGCA GCACCCATGCTGCGTGCGGCTGGAGCAGGCCTGGGA GGAGGGCGGCATCCTGTACCTGCAGACGGAGCTGTG CGGGCCCAGCCTGCAGCAACACTGTGAGGCCTGGGG TGCCAGCCTGCCTGAGGCCCAGGTCTGGGGCTACCTG CGGGACACGCTGCTTGCCCTGGCCCATCTGCACAGCC AGGGCCTGGTGCACCTTGATGTCAAGCCTGCCAACAT CTTCCTGGGGCCCCGGGGCCGCTGCAAGCTGGGTGA CTTCGGACTGCTGGTGGAGCTGGGTACAGCAGGAGC TGGTGAGGTCCAGGAGGGAGACCCCCGCTACATGGC CCCCGAGCTGCTGCAGGGCTCCTATGGGACAGCAGC GGATGTGTTCAGTCTGGGCCTCACCATCCTGGAAGTG GCATGCAACATGGAGCTGCCCCACGGTGGGGAGGGC TGGCAGCAGCTGCGCCAGGGCTACCTGCCCCCTGAGT TCACTGCCGGTCTGTCTTCCGAGCTGCGTTCTGTCCTT GTCATGATGCTGGAGCCAGACCCCAAGCTGCGGGCC ACGGCCGAGGCCCTGCTGGCACTGCCTGTGTTGAGG CAGCCGCGGGCCTGGGGTGTGCTGTGGTGCATGGCA GCGGAGGCCCTGAGCCGAGGGTGGGCCCTGTGGCA GGCCCTGCTTGCCCTGCTCTGCTGGCTCTGGCATGGG CTGGCTCACCCTGCCAGCTGGCTACAGCCCCTGGGCC CGCCAGCCACCCCGCCTGGCTCACCACCCTGCAGTTT GCTCCTGGACAGCAGCCTCTCCAGCAACTGGGATGAC GACAGCCTAGGGCCTTCACTCTCCCCTGAGGCTGTCC TGGCCCGGACTGTGGGGAGCACCTCCACCCCCCGGA GCAGGTGCACACCCAGGGATGCCCTGGACCTAAGTG ACATCAACTCAGAGCCTCCTCGGGGCTCCTTCCCCTCC TTTGAGCCTCGGAACCTCCTCAGCCTGTTTGAGGACA CCCTAGACCCAACCTGA |
| **Seq. ID. 2*** | anti-PKMYT1 Silencer^{®} Pre-designed siRNA, sense | Synthetic construct | RNA | Other RNA | GUGGCAUGCAACAUGGAGCtt |
| **Seq. ID. 3*** | anti-PKMYT1 Silencer^{®} Pre-designed siRNA, antisense | Synthetic construct | RNA | Other RNA | GCUCCAUGUUGCAUGCCACtt |
| **Seq. ID. 4*** | Primer PKMYT_Hi ndIII_forw ard | Synthetic construct | DNA | Other DNA | 5'-AATAAGCTTCCATGCTAGAACGGCCTCCT-3' |
| **Seq. ID. 5*** | Primer PKMYT_Ba mHI_rever se | Synthetic construct | DNA | Other DNA | 5'-ATAGGATCCTCAGGTTGGGTCTAGGGT-3' |

| | | | | | |
|---|---|---|---|---|---|
| * Outside the subject-matter of the claims but useful for understanding the disclosure | | | | | |

**Table 5 - The following table describes the list of sequences described in the present application, for which an accession number is available.**

| **Seq. ID** | **Accession number** | **Sequence name** | **Organism name** | **Molecule type** | **Qualifier Molecule Type** |
|---|---|---|---|---|---|
| **Seq. ID. 6** | **NM_004203.5** (NCBI database) | PKMYT1 cDNA of isoform 1 | Homo sapiens | DNA | Genomic DNA |
| **Seq. ID. 7** | CCDS10486.1 (NCBI database) | PKMYT1-CDS | Homo sapiens | DNA | Genomic DNA |
| **Seq. ID. 8** | UniProt Accession Number Q99640-1 | PKMYT1- isoform 1 | Homo sapiens | AA (amino acid) | protein |
| **Seq. ID. 9** | UniProt Accession Number Q99640-2 | PKMYT1- isoform 2 | Homo sapiens | AA (amino acid) | protein |
| **Seq. ID. 10** | UniProt Accession Number Q99640-3 | PKMYT1- isoform 3 | Homo sapiens | AA (amino acid) | protein |
| **Seq. ID. 11** | UniProt Accession Number Q99640-4 | PKMYT1- isoform 4 | Homo sapiens | AA (amino acid) | protein |

**Table 6 - The following table describes the residues of the sequences listed above**

| **Seq. ID** | **Residues** |
|---|---|
| **Seq. ID. 6** | GCGCGTTCACGGGCCGTTCCCCCTCACGGGAGTCCTCCGCCCGGGCGTCCGGAACAGTCGACGGC AGACTCCGGCCCGCTGAGCCACCCGAGGGGTCCCGTGGCCTCCGCGGACCCGGAATCTGGGCCCT CGCGGACCCGCGCCCCGCCCAGTCGCCCCAGGGCTTCCCCACACCCACGGAGTGAAGTCAGCCGC GGCCCTGCCTGGGAGGAACTTACCGTCTACCGGGAAAGGTGGCCAGCAGATGTGTCGGGCCTGGT GAGAGGGTGAGGCGAGACGGCCCGATCGCCCAGGGCCCCGGAAGCTGCGGAGGTCACCCCCGCC TGGCCTTAGCTCAGGGACACCCTGGATTCACGTGGGAGCCCCTGCTCCTGCCTCCCCCGTCCCACCA CTGAGGCTGTTGGGCCAGGCCAGTCATGCTAGAACGGCCTCCTGCACTGGCCATGCCCATGCCCAC GGAGGGCACCCCGCCACCTCTGAGTGGCACCCCCATCCCAGTCCCAGCCTACTTCCGCCACGCAGA ACCTGGATTCTCCCTCAAGAGGCCCAGGGGGCTCAGCCGGAGCCTCCCACCTCCGCCCCCTGCCAA GGGCAGCATTCCCATCAGCCGCCTCTTCCCTCCTCGGACCCCAGGCTGGCACCAGCTGCAGCCCCG GCGGGTGTCATTCCGGGGCGAGGCCTCAGAGACTCTGCAGAGCCCTGGGTATGACCCAAGCCGGC CAGAGTCCTTCTTCCAGCAGAGCTTCCAGAGGCTCAGCCGCCTGGGCCATGGCTCCTACGGAGAG GTCTTCAAGGTGCGCTCCAAGGAGGACGGCCGGCTCTATGCGGTAAAGCGTTCCATGTCACCATTC CGGGGCCCCAAGGACCGGGCCCGCAAGTTGGCCGAGGTGGGCAGCCACGAGAAGGTGGGGCAG CACCCATGCTGCGTGCGGCTGGAGCAGGCCTGGGAGGAGGGCGGCATCCTGTACCTGCAGACGG AGCTGTGCGGGCCCAGCCTGCAGCAACACTGTGAGGCCTGGGGTGCCAGCCTGCCTGAGGCCCAG GTCTGGGGCTACCTGCGGGACACGCTGCTTGCCCTGGCCCATCTGCACAGCCAGGGCCTGGTGCA CCTTGATGTCAAGCCTGCCAACATCTTCCTGGGGCCCCGGGGCCGCTGCAAGCTGGGTGACTTCGG ACTGCTGGTGGAGCTGGGTACAGCAGGAGCTGGTGAGGTCCAGGAGGGAGACCCCCGCTACATG GCCCCCGAGCTGCTGCAGGGCTCCTATGGGACAGCAGCGGATGTGTTCAGTCTGGGCCTCACCATC CTGGAAGTGGCATGCAACATGGAGCTGCCCCACGGTGGGGAGGGCTGGCAGCAGCTGCGCCAGG GCTACCTGCCCCCTGAGTTCACTGCCGGTCTGTCTTCCGAGCTGCGTTCTGTCCTTGTCATGATGCT GGAGCCAGACCCCAAGCTGCGGGCCACGGCCGAGGCCCTGCTGGCACTGCCTGTGTTGAGGCAGC CGCGGGCCTGGGGTGTGCTGTGGTGCATGGCAGCGGAGGCCCTGAGCCGAGGGTGGGCCCTGTG GCAGGCCCTGCTTGCCCTGCTCTGCTGGCTCTGGCATGGGCTGGCTCACCCTGCCAGCTGGCTACA GCCCCTGGGCCCGCCAGCCACCCCGCCTGGCTCACCACCCTGCAGTTTGCTCCTGGACAGCAGCCT |
| | CTCCAGCAACTGGGATGACGACAGCCTAGGGCCTTCACTCTCCCCTGAGGCTGTCCTGGCCCGGAC TGTGGGGAGCACCTCCACCCCCCGGAGCAGGTGCACACCCAGGGATGCCCTGGACCTAAGTGACA TCAACTCAGAGCCTCCTCGGGGCTCCTTCCCCTCCTTTGAGCCTCGGAACCTCCTCAGCCTGTTTGA GGACACCCTAGACCCAACCTGAGCCCCAGACTCTGCCTCTGCACTTTTAACCTTTTATCCTGTGTCTC TCCCGTCGCCCTTGAAAGCTGGGGCCCCTCGGGAACTCCCATGGTCTTCTCTGCCTGGCCGTGTCTA ATAAAAAGTATTTGAACCTTGGGAGCACCCAA |
| **Seq. ID. 7** | ATGCTAGAACGGCCTCCTGCACTGGCCATGCCCATGCCCACGGAGGGCACCCCGCCACCTCTGAGT GGCACCCCCATCCCAGTCCCAGCCTACTTCCGCCACGCAGAACCTGGATTCTCCCTCAAGAGGCCCA GGGGGCTCAGCCGGAGCCTCCCACCTCCGCCCCCTGCCAAGGGCAGCATTCCCATCAGCCGCCTCT TCCCTCCTCGGACCCCAGGCTGGCACCAGCTGCAGCCCCGGCGGGTGTCATTCCGGGGCGAGGCC TCAGAGACTCTGCAGAGCCCTGGGTATGACCCAAGCCGGCCAGAGTCCTTCTTCCAGCAGAGCTTC CAGAGGCTCAGCCGCCTGGGCCATGGCTCCTACGGAGAGGTCTTCAAGGTGCGCTCCAAGGAGGA CGGCCGGCTCTATGCGGTAAAGCGTTCCATGTCACCATTCCGGGGCCCCAAGGACCGGGCCCGCA AGTTGGCCGAGGTGGGCAGCCACGAGAAGGTGGGGCAGCACCCATGCTGCGTGCGGCTGGAGCA GGCCTGGGAGGAGGGCGGCATCCTGTACCTGCAGACGGAGCTGTGCGGGCCCAGCCTGCAGCAA CACTGTGAGGCCTGGGGTGCCAGCCTGCCTGAGGCCCAGGTCTGGGGCTACCTGCGGGACACGCT GCTTGCCCTGGCCCATCTGCACAGCCAGGGCCTGGTGCACCTTGATGTCAAGCCTGCCAACATCTTC CTGGGGCCCCGGGGCCGCTGCAAGCTGGGTGACTTCGGACTGCTGGTGGAGCTGGGTACAGCAG GAGCTGGTGAGGTCCAGGAGGGAGACCCCCGCTACATGGCCCCCGAGCTGCTGCAGGGCTCCTAT GGGACAGCAGCGGATGTGTTCAGTCTGGGCCTCACCATCCTGGAAGTGGCATGCAACATGGAGCT GCCCCACGGTGGGGAGGGCTGGCAGCAGCTGCGCCAGGGCTACCTGCCCCCTGAGTTCACTGCCG GTCTGTCTTCCGAGCTGCGTTCTGTCCTTGTCATGATGCTGGAGCCAGACCCCAAGCTGCGGGCCA CGGCCGAGGCCCTGCTGGCACTGCCTGTGTTGAGGCAGCCGCGGGCCTGGGGTGTGCTGTGGTGC ATGGCAGCGGAGGCCCTGAGCCGAGGGTGGGCCCTGTGGCAGGCCCTGCTTGCCCTGCTCTGCTG GCTCTGGCATGGGCTGGCTCACCCTGCCAGCTGGCTACAGCCCCTGGGCCCGCCAGCCACCCCGCC TGGCTCACCACCCTGCAGTTTGCTCCTGGACAGCAGCCTCTCCAGCAACTGGGATGACGACAGCCT AGGGCCTTCACTCTCCCCTGAGGCTGTCCTGGCCCGGACTGTGGGGAGCACCTCCACCCCCCGGAG CAGGTGCACACCCAGGGATGCCCTGGACCTAAGTGACATCAACTCAGAGCCTCCTCGGGGCTCCTT CCCCTCCTTTGAGCCTCGGAACCTCCTCAGCCTGTTTGAGGACACCCTAGACCCAACCTGA |
| **Seq. ID. 8** | MLERPPALAMPMPTEGTPPPLSGTPIPVPAYFRHAEPGFSLKRPRGLSRSLPPPPPAKGSIPISRLFPPRT PGWHQLQPRRVSFRGEASETLQSPGYDPSRPESFFQQSFQRLSRLGHGSYGEVFKVRSKEDGRLYAVK RSMSPFRGPKDRARKLAEVGSHEKVGQHPCCVRLEQAWEEGGILYLQTELCGPSLQQHCEAWGASLP EAQVWGYLRDTLLALAHLHSQGLVHLDVKPANIFLGPRGRCKLGDFGLLVELGTAGAGEVQEGDPRY MAPELLQGSYGTAADVFSLGLTILEVACNMELPHGGEGWQQLRQGYLPPEFTAGLSSELRSVLVMML EPDPKLRATAEALLALPVLRQPRAWGVLWCMAAEALSRGWALWQALLALLCWLWHGLAHPASWLQ PLGPPATPPGSPPCSLLLDSSLSSNWDDDSLGPSLSPEAVLARTVGSTSTPRSRCTPRDALDLSDINSEPP RGSFPSFEPRNLLSLFEDTLDPT |
| **Seq. ID. 9** | MLERPPALAMPMPTEGTPPPLSGTPIPVPAYFRHAEPGFSLKRPRGLSRSLPPPPPAKGSIPISRLFPPRT PGWHQLQPRRVSFRGEASETLQSPGYDPSRPESFFQQSFQRLSRLGHGSYGEVFKVRSKEDGRLYAVK RSMSPFRGPKDRARKLAEVGSHEKVGQHPCCVRLEQAWEEGGILYLQTELCGPSLQQHCEAWGASLP EAQVWGYLRDTLLALAHLHSQGLVHLDVKPANIFLGPRGRCKLGDFGLLVELGTAGAGEVQEGDPRY MAPELLQGSYGTAADVFSLGLTILEVACNMELPHGGEGWQQLRQGYLPPEFTAGLSSELRSVLVMML EPDPKLRATAEALLALPVLRQPRAWGVLWCMAAEALSRGWALWQALLALLCWLWHGLAHPASWLQ PLGPPATPPGSPPCSLLLDSSLSSNWDDDSLGPGHPPCLACPPAGLHSPLRLSWPGLWGAPPPPGAGA HPGMPWT |
| **Seq. ID. 10** | MPMPTEGTPPPLSGTPIPVPAYFRHAEPGFSLKRPRGLSRSLPPPPPAKGSIPISRLFPPRTPGWHQLQP RRVSFRGEASETLQSPGYDPSRPESFFQQSFQRLSRLGHGSYGEVFKVRSKEDGRLYAVKRSMSPFRGP KDRARKLAEVGSHEKVGQHPCCVRLEQAWEEGGILYLQTELCGPSLQQHCEAWGASLPEAQVWGYL RDTLLALAHLHSQGLVHLDVKPANIFLGPRGRCKLGDFGLLVELGTAGAGEVQEGDPRYMAPELLQGS YGTAADVFSLGLTILEVACNMELPHGGEGWQQLRQGYLPPEFTAGLSSELRSVLVMMLEPDPKLRATA EALLALPVLRQPRAWGVLWCMAAEALSRGWALWQALLALLCWLWHGLAHPASWLQPLGPPATPPG SPPCSLLLDSSLSSNWDDDSLGPSLSPEAVLARTVGSTSTPRSRCTPRDALDLSDINSEPPRGSFPSFEPR NLLSLFEDTLDPT |
| **Seq. ID. 11** | MLERWHQLQPRRVSFRGEASETLQSPGYDPSRPESFFQQSFQRLSRLGHGSYGEVFKVRSKEDGRLYA VKRSMSPFRGPKDRARKLAEVGSHEKVGQHPCCVRLEQAWEEGGILYLQTELCGPSLQQHCEAWGAS LPEAQVWGYLRDTLLALAHLHSQGLVHLDVKPANIFLGPRGRCKLGDFGLLVELGTAGAGEVQEGDPR YMAPELLQGSYGTAADVFSLGLTILEVACNMELPHGGEGWQQLRQGYLPPEFTAGLSSELRSVLVMM LEPDPKLRATAEALLALPVLRQPRAWGVLWCMAAEALSRGWALWQALLALLCWLWHGLAHPASWL QPLGPPATPPGSPPCSLLLDSSLSSNWDDDSLGPSLSPEAVLARTVGSTSTPRSRCTPRDALDLSDINSEP PRGSFPSFEPRNLLSLFEDTLDPT |

The cloned pEGFP-PKMYT1 plasmid was used in a gain-in-function assay, where SH-SY5Y cells were seeded at ^{~}3.0 x 10⁴/cm². Cells were transfected with pEGFP-PKMYT1 24 hours after plating using Turbofect (ThermoFisher), according to the supplier's protocol. On the same day, the medium was changed to the differentiation medium with 10 µM RA and 1% FBS. Cell lysates were collected in 1% SDS 48h after transfection (day 2 of the neuronal differentiation program).

### SDS-PAGE and immunoblotting

Lysates collected in 1% SDS were sonicated and boiled for 10 min. Protein content was determined using the Pierce^{™} bicinchoninic acid (BCA) Protein Assay Kit (Pierce Biotechnology). Cell lysates (mass-normalized by protein content) were resolved on a 10% or a 5-20% gradient sodium dodecyl sulphate-polyacrylamide gel electrophoresis (SDS-PAGE) in Tris-Glycine buffer and later electro-transferred onto nitrocellulose membranes. Gels were loaded with 20-70 µg of protein (depending on total protein available per replica). The membranes were first incubated with Ponceau S (Sigma-Aldrich, 0.1% [w/v] in 5% acetic acid) to serve as a loading control since common cytoskeleton markers are inappropriate loading controls for cell differentiation assays. Membranes were blocked with 5% non-fat dry milk/TBS-T for 2h at room temperature (RT), and further incubated with the primary antibodies in 3% BSA/TBS-T under agitation for 2h at RT, and ON at 4°C. The primary antibodies used were: mouse anti-PKMYT1 (1:1000; Invitrogen, MA5-25034) and mouse anti-βIII-Tubulin (1:4000; Sigma-Aldrich, T8578). The secondary antibody HRP-linked anti-mouse IgG in 3% non-fat dry milk/TBS-T (1:3000 or 1:5000; Cell Signaling, 7076S) was incubated for 1h at RT under agitation. Proteins were detected by enhanced chemiluminescence using the Amersham ECL Prime or Amersham ECL Select detection reagents (Cytiva, RPN2236 and RPN2235). The protein signal was scanned in a ChemiDoc Imaging System (Bio-Rad) and analyzed with ImageLab Software (Bio-Rad). Protein level quantification was corrected to the total loading determined by the Ponceau staining.

### PKMYT1 bioinformatic analyses of post-translational modifications

The Eukaryotic Linear Motif (ELM) software in the ExPASy portal was used to obtain a list of the predicted short linear motifs (SLiMs) for PKMYT1 by entering the FASTA sequence of its canonical form (UniProt Accession Number Q99640-1), to identify PKMYT1 post-translational modifications that explain variations in PKMYT1 molecular weight.

### PKMYT1 immunofluorescence analysis

For immunocytochemistry (ICC) analysis, cells grown on coverslips were fixed with 4% paraformaldehyde for 20 min, further permeabilized for 10 min with 0.2% Triton X-100/PBS and blocked for 30 min with 1% BSA/PBS-T (0.1% Tween 20). Primary antibodies diluted in blocking solution were incubated for 2 h at RT: mouse anti-PKMYT1 (1:100) or mouse anti-βIII-Tubulin (1:500). Secondary antibodies anti-mouse IgG Alexa Fluor^{™} 488 or anti-mouse Alexa Fluor^{™} 594 (1:300; Invitrogen, A11001 and A11005) were incubated for 1 h at RT in the dark. The coverslips were mounted on a microscope glass slide with DAPI-containing Vectashield antifading reagent (Vector) and the fluorescence images were observed in a Zeiss LSM 880 confocal microscope (Zeiss).

### Morphometric analyses of PhC and fluorescence microphotographs

PhC and immunofluorescence microphotographs were both analyzed using Fiji ImageJ v1.53t software. NeuronRead v1.2 macro for ImageJ was used to measure the total neuritic length and score cell number in PhC microphotographs, using median filter radius and morph filter radius set to 3, and the Otsu threshold detection mode. Regions of interest were selected manually to avoid false positives by detecting debris, shadows, and ripples. NeuronJ v1.4.3 plugin for ImageJ was used to measure neurites in GFP-fluorescing cells in immunofluorescence microphotographs of pEGFP vector alone and pEGFP-PKMYT1 transfected cells. Before measuring, the PKMYT1 signal was contrasted and merged with the βIII-Tubulin signal and saved in 8-bit images. The DAPI staining was used for cell scoring.

### Analysis of PKMYT1 mRNA in rat DRG neurons 24h upon non-regenerative and regenerative lesions

*Animal Models, Cryo-sectioning, and Laser Capture Microdissection of Dorsal Root Ganglion (DRG) neurons.* Two neuronal lesion paradigms were inflicted on adult rats: a) Central neuropathology (spinal cord injury; SCI) by complete axotomy of the spinal cord at the T8-9 level; b) Peripheral neuropathology (peripheral nerve injury; PNI) by sciatic nerve transection. The affected DRG tissues (L4, L5, L6) were collected 24 hours after lesion, immediately frozen in Methyl Butane, and stored at -80°C. The collected tissues were embedded with optimal cutting temperature (OCT) cryo-compound (Tissue-Tek, Sakura) and further cryo-sectioned in Glass or Membrane PEN-slides with 16 µm. Following, the neuronal cell bodies were collected by Laser Capture Microdissection (Zeiss, PALM MicroBeam LCM Laser Dissection System/AXIO Observer Microscope).

*RNA Extraction and Sequencing.* Total RNA extraction was performed with RNeasy Micro kit (Qiagen) according to the manufacturer protocol. DNAse treatment was applied. The yield and integrity of total isolated RNA from microdissected samples were measured using a 2100 Bioanalyzer (Agilent Technologies). For RNA sequencing, cDNA was first prepared using the SMART-Seq^{®} v4 Ultra^{®} Low Input RNA Kit (Takara Bio), and the libraries were prepared using Nextera XT DNA (Illumina). Samples were then sequenced in a NextSeq550 sequencer (Illumina).

*Analysis of Genes Differential Expression.* Initial quality checking of the raw reads was performed with FASTQC v0.11.7; Cutadapt v2.7 was subsequently used to remove the adapter contamination and Trimmomatic v0.36 was used to improve reads' quality. The reads were mapped against the *Rattus norvegicus* reference genome (Rn6.0) using Hisat v2.2.0 with default options. Later, the mapped reads were tracked to genome annotation (Rn6.0) and counted using Stringtie v2.1.1 (Pertea, 2015) with non-strand specific library type. The raw counts were submitted to an in-house pipeline using the DESeq2 R package (R v4.0.4; RStudio v1.4.1106). Genes with less than 5 summed raw counts in all samples or in all samples except one, were filtered out before further analysis. Normalization with housekeeping genes, and RIN and Batch adjustment were applied (when necessary). The model used tested for interaction between conditions (Injury vs Sham). Additional filters were added to remove outlier genes: the command rowSums(counts(dds) >= 10) >= X (X is the max(table(interaction group)) also removed genes with counts in only one sample, considering only the samples used for the statistical test. After the normalization step, exploratory and differential gene expression analysis was performed. Significant Differentially Expressed Genes (DEGs), like PKMYT1, were identified by comparing injury samples vs sham samples, in both types of injury (PNI and SCI) (p-value < 0.05; log₂fold change threshold = 0).

### Bioinformatic comparative analysis of transcriptomic studies in spinal cord naturally regenerating animals

*Data mining.* Different transcriptomic studies in vertebrate species that can naturally regenerate their spinal cords were comparatively analyzed to identify putative regeneration-associated genes (RAGs) behind their innate ability to fully restore lost functions after acute SCI. Data were searched in transcriptomic databases, like Gene Expression Omnibus (GEO), ArrayExpress, and Expression Atlas, using the keywords: "spinal cord" OR "spinal cord injury" OR "spinal cord lesion" OR "neuron". The search was extended to find transcriptomic studies in PubMed using the same keywords. Results were filtered by species according to their regeneration capabilities. These searches retrieved studies on *Danio rerio* (zebrafish), *Petromyzon marinus* (sea lamprey), *Ambystoma mexicanum* (axolotl), *Xenopus laevis* (African clawed toad) and *Xenopus tropicalis* (tropical clawed frog tadpoles). The transcriptomic data and studies obtained were analyzed regarding the applied RNA methodology (microarray or RNA sequencing), the injury procedure, the application of a treatment or not, the local of sample collection, collection time points and the number of replicates. Of eight studies found, three were excluded from the comparative analysis due to: a) missing a control/sham-operated group to compare with an SCI-operated group or with <3 replicates (PMID: 22085734); b) test of a pharmaceutical treatment (PMID: 26477559); c) unpublished data at the time of analysis (GEO Accession GSE56842).

### Processing and analysis of retrieved data.

When the available data of the remaining five studies **(Table 2)** consisted of a list solely with the probe identifiers and respective intensities, data from each study was organized into a table listing the transcripts' identifiers and column groups considering each timepoint measured and the biological states (control/sham or induced SCI), containing at least three biological replicates. The differential expression was further calculated using the 'Analyse Data' tool from Reactome v81, by selecting 'Analyse gene expression' and choosing the method of analysis 'Camera', which uses a gene analysis algorithm as in the R 'limma' package for microarray data analysis. Data for analysis (normalized microarray) was uploaded and the biological states and timepoints to be compared were defined (control/sham vs induced SCI, for each time point). The analysis returned a list of UniProt Accession Codes of the human orthologues corresponding to the probes' identifiers, which were translated to their gene names using Synergizer, a repository of genes and their protein identifier synonyms. Statistically significant DEGs and their respective log₂ fold changes (FC) were obtained or calculated from the transcriptomic raw datasets.

### Data analysis and statistics

Fold changes (FC) were calculated by comparing the values of each time point or condition analysed to the levels of the first control time point analysed within each assay (taken as 1.0 or 100%), and averaged between independent replicates. Results are presented as average and standard deviation of independent biological replicates (N, indicated in each figure). Statistical analyses were conducted using the GraphPad Prism 9.3.0 software; confidence intervals of 95% were considered. Variance analysis was performed using one-way ANOVA and Dunnett's multiple comparisons test (for comparisons between different time points in the confluency assay), or two-way ANOVA and Sidak's multiple comparisons test (for comparison between different conditions in time-dependent profiles of the neuronal differentiation time-course). Variance analysis between control and tested conditions at the same time point was performed using the two-tailed unpaired t test.

The term "comprising" whenever used in this document is intended to indicate the presence of stated features, integers, steps, components, but not to preclude the presence or addition of one or more other features, integers, steps, components, or groups thereof.

The disclosure should not be seen in any way restricted to the embodiments described and a person with ordinary skill in the art will foresee many possibilities to modifications thereof.

The following claims further set out particular embodiments of the disclosure.

### REFERENCES

1. Wang KC, Koprivica V, Kim JA, et al. Oligodendrocyte-myelin glycoprotein is a Nogo receptor ligand that inhibits neurite outgrowth. Nature. 2002;417(6892):941-944. doi:10.1038/nature00867
2. Schwab JM, Failli V, Chédotal A. Injury-related dynamic myelin/oligodendrocyte axon-outgrowth inhibition in the central nervous system. The Lancet. 2005;365(9476):2055-2057. doi:10.1016/S0140-6736(05)66699-8
3. Ahuja CS, Wilson JR, Nori S, et al. Traumatic spinal cord injury. Nat Rev Dis Primers. 2017;3(1):17018. doi:10.1038/nrdp.2017.18
4. Ashammakhi N, Kim HJ, Ehsanipour A, et al. Regenerative Therapies for Spinal Cord Injury. Tissue Engineering - Part B: Reviews. 2019;25(6):471-491. doi:10.1089/ten.teb.2019.0182
5. Asquith CRM, Laitinen T, East MP. PKMYT1: a forgotten member of the WEE1 family. Nature reviews Drug discovery. 2020;19(3):157. doi:10.1038/d41573-019-00202-9
6. Platzer C, Najjar A, Rohe A, Erdmann F, Sippl W, Schmidt M. Identification of PKMYT1 inhibitors by screening the GSK published protein kinase inhibitor set I and II. Bioorganic and Medicinal Chemistry. 2018;26(14):4014-4024. doi: 10.1016/j.bmc.2018.06.027
7. Liu F, Rothblum-Oviatt C, Ryan CE, Piwnica-Worms H. Overproduction of Human Myt1 Kinase Induces a G2 Cell Cycle Delay by Interfering with the Intracellular Trafficking of Cdc2-Cyclin B1 Complexes. Molecular and Cellular Biology. 1999;19(7):5113-5123. doi:10.1128/mcb.19.7.5113
8. Booher RN, Holman PS, Fattaey A. Human Myt1 is a cell cycle-regulated kinase that inhibits Cdc2 but not Cdk2 activity. Journal of Biological Chemistry. 1997;272(35):22300-22306. doi: 10.107 4/jbc.272.35.22300
9. Ruiz EJ, Vilar M, Nebreda AR. A Two-Step Inactivation Mechanism of Myt1 Ensures CDK1/Cyclin B Activation and Meiosis I Entry. Current Biology. 2010;20(8):717-723. doi:10.1016/j.cub.2010.02.050
10. Strauss B, Harrison A, Coelho PA, Yata K, Zernicka-Goetz M, Pines J. Cyclin B1 is essential for mitosis in mouse embryos, and its nuclear export sets the time for mitosis. Journal of Cell Biology. 2018;217(1):179-193. doi: 10.1083/jcb.201612147
11. Szmyd R, Niska-Blakie J, Diril MK, et al. Premature activation of Cdk1 leads to mitotic events in S phase and embryonic lethality. Oncogene. 2019;38(7):998-1018. doi:10.1038/s41388-018-0464-0
12. Ma L, Shen YQ, Khatri HP, Schachner M. The asparaginyl endopeptidase legumain is essential for functional recovery after spinal cord injury in adult zebrafish. PLoS ONE. 2014;9(4). doi:10.1371/journal.pone.0095098
13. Herman PE, Papatheodorou A, Bryant SA, et al. Highly conserved molecular pathways, including Wnt signaling, promote functional recovery from spinal cord injury in lampreys. Scientific Reports. 2018;8(1). doi:10.1038/s41598-017-18757-1
14. Lee-Liu D, Moreno M, Almonacid LI, et al. Genome-wide expression profile of the response to spinal cord injury in Xenopus laevis reveals extensive differences between regenerative and non-regenerative stages. Neural Development. 2014;9(1):12. doi:10.1186/1749-8104-9-12
15. Guo Y, Ma L, Cristofanilli M, Hart RP, Hao A, Schachner M. Transcription factor Sox11b is involved in spinal cord regeneration in adult zebrafish. Neuroscience. 2011;172:329-341. doi:10.1016/j.neuroscience.2010.10.026
16. Hui SP, Sengupta D, Lee SGP, et al. Genome wide expression profiling during spinal cord regeneration identifies comprehensive cellular responses in zebrafish. PLoS ONE. 2014;9(1). doi:10.1371/journal.pone.0084212

## Claims

1. An isolated or artificial nucleotide sequence encoding the protein PKMYT1, wherein the sequence encoding the protein PKMYT1 is identical to a sequence selected from a list consisting of: SEQ. ID. 1, SEQ. ID. 6, SEQ. ID. 7; for use in the treatment or regeneration of neurons following traumatic injuries on the central or peripheral nervous system.

2. The isolated or artificial nucleotide sequence for use according to the previous claim for use in the treatment or regeneration of neurons following traumatic injuries on the central nervous system.

3. The isolated or artificial nucleotide sequence according to any of the previous claims for use in the treatment of spinal cord injury.

4. The isolated or artificial nucleotide sequence for use according to any of the previous claims, wherein said sequence is to be administered directly by in situ injection, by intravascular injection, intravenous injection, intranasal injection, intraventricular injection or intrathecal injection means; preferably by in situ injection.

5. A vector or construct comprising the isolated or artificial nucleotide sequence as described in any of the previous claims for use in the treatment or regeneration of neurons following traumatic injuries on the central or peripheral nervous system; preferably for use in the treatment of spinal cord injury.

6. The vector for use according to the previous claim, wherein the vector is selected from the group consisting of: plasmid, liposome, dendrimer, nanoparticle vector, virus vector, preferably adenovirus, lentivirus, retrovirus, herpesvirus and Adeno-Associated Virus; preferably Adeno-Associated Virus or plasmid.

7. Host cell comprising the vector as described in any of the previous claims 5-6 for use in the treatment or regeneration of neurons following traumatic injuries on the central or peripheral nervous system; preferably central nervous systems; more preferably for use in the treatment of spinal cord injury.

8. Protein PKMYT1 comprising an amino acid sequence identical to a sequence selected from a list consisting of: SEQ. ID. 8; SEQ. ID. 9; SEQ. ID. 10, SEQ. ID. 11, or mixtures thereof, for use in the treatment or regeneration of neurons following traumatic injuries on the central or peripheral nervous system; preferably central nervous systems; more preferably for use in the treatment of spinal cord injury.

9. A pharmaceutical composition comprising a therapeutically effective amount of an isolated or artificial nucleotide sequence as described in any of the previous claims 1-4, or a vector as described in any of the previous claims 5-6, or a host cell as described in the previous claim 7, or a protein as described in the previous claim 8, or combinations thereof, for use in the treatment or regeneration of neurons following traumatic injuries on the central or peripheral nervous system; preferably central nervous systems; more preferably for use in the treatment of spinal cord injury.

10. A liposome, exosome or nanoparticle comprising an isolated or artificial nucleotide sequence as described in any of the previous claims 1-4, or a vector as described in any of the previous claims 5-6, or a host cell as described in the previous claim 7, or a protein as described in the previous claim 8, or combinations thereof, for use in the treatment or regeneration of neurons following traumatic injuries on the central or peripheral nervous system.

11. The liposome, exosome or nanoparticle according to the previous claim for use in the treatment of spinal cord injury.

12. A kit comprising an isolated or artificial nucleotide sequence as described in any of the previous claims 1-4, or a vector as described in any of the previous claims 5-6, or a host cell as described in the previous claim 7, or a protein as described in the previous claim 8, or combinations thereof, for use in the treatment or regeneration of neurons following traumatic injuries on the central or peripheral nervous system; preferably central nervous systems; more preferably for use in the treatment of spinal cord injury.

## Patentansprüche

1. Eine isolierte oder künstliche Nukleotidsequenz, die das Protein PKMYT1 codiert, wobei die Sequenz, die das Protein PKMYT1 codiert, identisch ist mit einer Sequenz, die aus einer Liste ausgewählt ist, bestehend aus: SEQ. ID. 1, SEQ. ID. 6, SEQ. ID. 7; zur Verwendung bei der Behandlung oder Regeneration von Neuronen nach traumatischen Verletzungen des zentralen oder peripheren Nervensystems.

2. Die isolierte oder künstliche Nukleotidsequenz zur Verwendung gemäß dem vorhergehenden Anspruch zur Verwendung bei der Behandlung oder Regeneration von Neuronen nach traumatischen Verletzungen des zentralen Nervensystems.

3. Die isolierte oder künstliche Nukleotidsequenz gemäß einem der vorhergehenden Ansprüche zur Verwendung bei der Behandlung von Rückenmarksverletzungen.

4. Die isolierte oder künstliche Nukleotidsequenz zur Verwendung gemäß einem der vorhergehenden Ansprüche, wobei die genannte Sequenz direkt durch In-situ-Injektion, durch intravaskuläre Injektion, intravenöse Injektion, intranasale Injektion, intraventrikuläre Injektion oder intrathekale Injektion verabreicht wird; vorzugsweise durch In-situ-Injektion.

5. Ein Vektor oder Konstrukt, das die isolierte oder künstliche Nukleotidsequenz gemäß einem der vorhergehenden Ansprüche umfasst, zur Verwendung bei der Behandlung oder Regeneration von Neuronen nach traumatischen Verletzungen des zentralen oder peripheren Nervensystems; vorzugsweise zur Verwendung bei der Behandlung von Rückenmarksverletzungen.

6. Der Vektor zur Verwendung gemäß dem vorhergehenden Anspruch, wobei der Vektor aus der Gruppe ausgewählt ist, die besteht aus: Plasmid, Liposom, Dendrimer, Nanopartikel-Vektor, Virus-Vektor, vorzugsweise Adenovirus, Lentivirus, Retrovirus, Herpesvirus und Adeno-assoziiertes Virus; vorzugsweise Adeno-assoziiertes Virus oder Plasmid.

7. Wirtszelle, die den in einem der vorhergehenden Ansprüche 5-6 beschriebenen Vektor umfasst, zur Verwendung bei der Behandlung oder Regeneration von Neuronen nach traumatischen Verletzungen des zentralen oder peripheren Nervensystems; vorzugsweise des zentralen Nervensystems; besonders bevorzugt zur Verwendung bei der Behandlung von Rückenmarksverletzungen.

8. Protein PKMYT1, umfassend eine Aminosäuresequenz, die identisch ist mit einer Sequenz, ausgewählt aus einer Liste bestehend aus: SEQ. ID. 8; SEQ. ID. 9; SEQ. ID. 10, SEQ. ID. 11 oder Mischungen davon, zur Verwendung bei der Behandlung oder Regeneration von Neuronen nach traumatischen Verletzungen des zentralen oder peripheren Nervensystems; vorzugsweise des zentralen Nervensystems; besonders bevorzugt zur Verwendung bei der Behandlung von Rückenmarksverletzungen.

9. Eine pharmazeutische Zusammensetzung, umfassend eine therapeutisch wirksame Menge einer isolierten oder künstlichen Nukleotidsequenz, wie in einem der vorhergehenden Ansprüche 1 - 4 beschrieben, oder ein Vektor, wie in einem der vorhergehenden Ansprüche 5 - 6 beschrieben, oder eine Wirtszelle, wie in dem vorhergehenden Anspruch 7 beschrieben, oder ein Protein, wie in dem vorhergehenden Anspruch 8 beschrieben, oder Kombinationen davon, zur Verwendung bei der Behandlung oder Regeneration von Neuronen nach traumatischen Verletzungen des zentralen oder peripheren Nervensystems; vorzugsweise des zentralen Nervensystems; besonders bevorzugt zur Verwendung bei der Behandlung von Rückenmarksverletzungen.

10. Ein Liposom, Exosom oder Nanopartikel, umfassend eine isolierte oder künstliche Nukleotidsequenz wie in einem der vorhergehenden Ansprüche 1 - 4 beschrieben, oder ein Vektor wie in einem der vorhergehenden Ansprüche 5 - 6 beschrieben, oder eine Wirtszelle wie in dem vorhergehenden Anspruch 7 beschrieben, oder ein Protein wie in dem vorhergehenden Anspruch 8 beschrieben, oder Kombinationen davon, zur Verwendung bei der Behandlung oder Regeneration von Neuronen nach traumatischen Verletzungen des zentralen oder peripheren Nervensystems.

11. Das Liposom, Exosom oder Nanopartikel gemäß dem vorhergehenden Anspruch zur Verwendung bei der Behandlung von Rückenmarksverletzungen.

12. Ein Kit, umfassend eine isolierte oder künstliche Nukleotidsequenz, wie in einem der vorhergehenden Ansprüche 1 - 4 beschrieben, oder ein Vektor, wie in einem der vorhergehenden Ansprüche 5 - 6 beschrieben, oder eine Wirtszelle, wie in dem vorhergehenden Anspruch 7 beschrieben, oder ein Protein, wie in dem vorhergehenden Anspruch 8 beschrieben, oder Kombinationen davon, zur Verwendung bei der Behandlung oder Regeneration von Neuronen nach traumatischen Verletzungen des zentralen oder peripheren Nervensystems; vorzugsweise des zentralen Nervensystems; vorzugsweise zur Verwendung bei der Behandlung von Rückenmarksverletzungen.

## Revendications

1. Une séquence nucléotidique isolée ou artificielle codant pour la protéine PKMYT1, dans laquelle la séquence codant pour la protéine PKMYT1 est identique à une séquence choisie parmi une liste comprenant: SEQ. ID. 1, SEQ. ID. 6, SEQ. ID. 7 ; destinée à être utilisée dans le traitement ou la régénération de neurones à la suite de lésions traumatiques du système nerveux central ou périphérique.

2. La séquence nucléotidique isolée ou artificielle destinée à être utilisée selon la revendication précédente pour le traitement ou la régénération de neurones à la suite de lésions traumatiques du système nerveux central.

3. La séquence nucléotidique isolée ou artificielle selon l'une quelconque des revendications précédentes, destinée à être utilisée dans le traitement des lésions de la moelle épinière.

4. La séquence nucléotidique isolée ou artificielle destinée à être utilisée selon l'une quelconque des revendications précédentes, dans laquelle ladite séquence doit être administrée directement par injection in situ, par injection intravasculaire, injection intraveineuse, injection intranasale, injection intraventriculaire ou injection intrathécale ; de préférence par injection *in situ.*

5. Un vecteur ou construction comprenant la séquence nucléotidique isolée ou artificielle telle que décrite dans l'une quelconque des revendications précédentes, destinée à être utilisée dans le traitement ou la régénération de neurones à la suite de lésions traumatiques du système nerveux central ou périphérique ; de préférence destinée à être utilisée dans le traitement des lésions de la moelle épinière.

6. Le vecteur destiné à être utilisé selon la revendication précédente, dans lequel le vecteur est choisi parmi le groupe comprenant: un plasmide, un liposome, un dendrimère, un vecteur à nanoparticules, un vecteur viral, de préférence un adénovirus, un lentivirus, un rétrovirus, un herpèsvirus et un virus adéno-associé; de préférence un virus adéno-associé ou un plasmide.

7. Cellule hôte comprenant le vecteur tel que décrit dans l'une quelconque des revendications 5 à 6 précédentes, destiné à être utilisé dans le traitement ou la régénération de neurones à la suite de lésions traumatiques du système nerveux central ou périphérique ; de préférence du système nerveux central ; plus préférablement pour une utilisation dans le traitement des lésions de la moelle épinière.

8. Protéine PKMYT1 comprenant une séquence d'acides aminés identique à une séquence choisie parmi une liste comprenant: SEQ. ID. 8 ; SEQ. ID. 9 ; SEQ. ID. 10, SEQ. ID. 11, ou des mélanges de celles-ci, destinée à être utilisée dans le traitement ou la régénération des neurones à la suite de lésions traumatiques du système nerveux central ou périphérique ; de préférence du système nerveux central ; plus préférablement pour une utilisation dans le traitement des lésions de la moelle épinière.

9. Une composition pharmaceutique comprenant une quantité thérapeutiquement efficace d'une séquence nucléotidique isolée ou artificielle telle que décrite dans l'une quelconque des revendications 1 - 4 précédentes, ou d'un vecteur tel que décrit dans l'une quelconque des revendications 5 - 6 précédentes, ou d'une cellule hôte telle que décrite dans la revendication 7 précédente, ou d'une protéine telle que décrite dans la revendication 8 précédente, ou des combinaisons de ceux-ci, destinée à être utilisée dans le traitement ou la régénération de neurones à la suite de lésions traumatiques du système nerveux central ou périphérique ; de préférence du système nerveux central ; plus préférablement pour une utilisation dans le traitement des lésions de la moelle épinière.

10. Un liposome, exosome ou nanoparticule comprenant une séquence nucléotidique isolée ou artificielle telle que décrite dans l'une quelconque des revendications 1 - 4 précédentes, ou un vecteur tel que décrit dans l'une quelconque des revendications 5 - 6 précédentes, ou une cellule hôte telle que décrite dans la revendication 7 précédente, ou une protéine telle que décrite dans la revendication 8 précédente, ou des combinaisons de ceux-ci, destinés à être utilisés dans le traitement ou la régénération de neurones à la suite de lésions traumatiques du système nerveux central ou périphérique.

11. Le liposome, l'exosome ou la nanoparticule selon la revendication précédente destiné à être utilisé dans le traitement des lésions de la moelle épinière.

12. Un Kit comprenant une séquence nucléotidique isolée ou artificielle telle que décrite dans l'une quelconque des revendications 1 - 4 précédentes, ou un vecteur tel que décrit dans l'une quelconque des revendications 5 - 6 précédentes, ou une cellule hôte telle que décrite dans la revendication 7 précédente, ou une protéine telle que décrite dans la revendication 8 précédente, ou des combinaisons de ceux-ci, destinés à être utilisés dans le traitement ou la régénération de neurones à la suite de lésions traumatiques du système nerveux central ou périphérique ; de préférence du système nerveux central ; plus préférablement pour une utilisation dans le traitement des lésions de la moelle épinière.
